(19) Europäisches Patentamt
European Patent Office
Office européen des brevets

(11) EP 1 684 870 B1

(12) FASCICULE DE BREVET EUROPEEN

(45) Date de publication et mention de la délivrance du brevet:
**02.01.2008 Bulletin 2008/01**

(21) Numéro de dépôt: **04786340.2**

(22) Date de dépôt: **20.08.2004**

(51) Int Cl.:
***A61Q 5/06*** (2006.01) ***A61K 8/04*** (2006.01)
***B65D 83/60*** (2006.01)

(86) Numéro de dépôt international:
**PCT/FR2004/002175**

(87) Numéro de publication internationale:
**WO 2005/020944 (10.03.2005 Gazette 2005/10)**

# (54) UTILISATION D UN COPOLYMERE A GRADIENT DE COMPOSITION DANS UN DISPOSITIF AEROSOL A DEUX COMPARTIMENTS ET DISPOSITIF AEROSOL LE COMPRENANT ASSOCIE A UN GAZ COMPRIME

VERWENDUNG EINES COPOLYMERS MIT GRADIERTER ZUSAMMENSETZUNG IN EINER AEROSOL-VORRICHTUNG MIT ZWEI FÄCHERN UND DIESES COPOLYMER UND EIN KOMPRIMIERTES GAS ENTHALTENDE AEROSOL-VORRICHTUNG

USE OF A COMPOSITIONALLY-GRADED COPOLYMER IN AN AEROSOL DEVICE COMPRISING TWO COMPARTMENTS, AND AEROSOL DEVICE COMPRISING SAID COPOLYMER AND A COMPRESSED GAS

(84) Etats contractants désignés:
**AT BE BG CH CY CZ DE DK EE ES FI FR GB GR HU IE IT LI LU MC NL PL PT RO SE SI SK TR**

(30) Priorité: **22.08.2003 FR 0310109**

(43) Date de publication de la demande:
**02.08.2006 Bulletin 2006/31**

(73) Titulaire: **L'ORÉAL**
**75008 Paris (FR)**

(72) Inventeurs:
- **GAWTREY, Jonathan**
**F-92100 Boulogne (FR)**
- **MOUGIN, Nathalie**
**F-75011 Paris (FR)**

(74) Mandataire: **Casalonga, Axel et al**
**Bureau Casalonga & Josse**
**Bayerstrasse 71/73**
**80335 München (DE)**

(56) Documents cités:
**EP-A- 1 444 974**
**WO-A-00/71607**
**WO-A-20/04055071**
**FR-A- 2 848 429**

- **MATYJASZEWSKI K. AND XIA J.: "Atom transfer radical polymerization" CHEM. REV., vol. 101, 2001, pages 2921-2990, XP002271873 cité dans la demande**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 684 870 B1

Printed by Jouve, 75001 PARIS (FR)

## Description

La présente invention concerne un dispositif aérosol à deux compartiments comprenant une composition de traitement capillaire particulière dans l'un et un gaz comprimé dans l'autre. Elle concerne aussi une utilisation d'un copolymère à gradient de composition dans des dispositifs aérosols à deux compartiments comprenant un gaz comprimé comme agent propulseur.

Les produits capillaires pour la mise en forme et/ou le maintien de la coiffure les plus répandus sur le marché de la cosmétique sont des compositions à pulvériser essentiellement constituées d'une solution le plus souvent alcoolique et d'un ou de plusieurs matériaux, généralement des résines polymères, appelés matériaux fixants, dont la fonction est de former des soudures entre les cheveux, en mélange avec divers adjuvants cosmétiques. Les matériaux fixants sont généralement des polymères fixants, c'est-à-dire des polymères filmogènes solubles ou dispersibles dans l'eau ou l'alcool tels que les copolymères acétate de vinyle/acide crotonique, les résines acryliques anioniques ou amphotères, les polyuréthanes...

Des compositions cosmétiques capillaires comprenant au moins un polymère filmogène à gradient qui comprend au moins deux monomères différents et présente un indice de polydispersité en masse (Ip) inférieur ou égal à 2,5, sont décrites dans EP 1 444 974 et FR 2 848 429 (documents tels que définis par l'article 54(3) CBE). Ces compositions cosmétiques peuvent être contenues dans des compositions aérosols dans lesquelles l'agent propulseur est choisi parmi le diméthyléther, les alcanes en $C_{3-5}$, le 1,1-difluoroéthane, les mélanges de diméthyléther et d'alcanes en $C_{3-5}$, et les mélanges de 1,1-difluoroéthane et de diméthyléther et/ou d'alcanes en $C_{3-5}$. D'autres compositions de soin capillaire, par exemple sous forme d'aérosol, comprenant des copolymères blocs de polysiloxane, sont décrites dans WO 00/71607, l'agent propulseur utilisé dans les aérosols pouvant être un produit chlorofluorocarboné, le diméthyléther, le propane, le n-butane ou l'isobutane.

Pour des raisons essentiellement écologiques, on cherche à réduire la quantité de composés organiques volatils (ou COV) présents dans la composition. Pour diminuer la quantité de COV et obtenir un dispositif aérosol à faible COV, les solvants organiques, comme l'éthanol et le diméthyléther, sont respectivement remplacés en partie par l'eau et un gaz comprimé.

Cependant, le remplacement de l'éthanol par de l'eau et du diméthyléther par un gaz comprimé tel que l'air, génère des inconvénients tels qu'une dégradation de la qualité du spray, des phénomènes de bouchage du dispositif aérosol et parfois, une perte des performances cosmétiques.

L'utilisation de polymères particuliers tels que des copolymères à gradient de composition, dans un milieu aqueux, permet d'éviter ces inconvénients, mais la formulation de ces polymères seuls avec du diméthyléther ou un autre gaz liquéfié comme agent propulseur, conduit à un blanchiment des cheveux.

Des polymères amphiphiles à gradient et leur procédé de synthèse sont décrits dans WO 2004/055071 (documents tels que défini par l'article 54(3) CBE).

La demanderesse a trouvé de manière surprenante que l'utilisation d'un dispositif aérosol à deux compartiments particulier dont l'agent propulseur est un gaz comprimé particulier associé éventuellement à au moins un gaz liquéfié, permettait d'éviter le blanchiment des cheveux et d'obtenir des produits capillaires pour la mise en forme et/ou le maintien de la coiffure à faible teneur en COV.

La présente invention a donc pour objet un dispositif aérosol à deux compartiments qui contient, dans un premier compartiment, une composition de traitement capillaire qui comprend, dans un milieu aqueux cosmétiquement acceptable, au moins un copolymère à gradient de composition tel que décrit ci-dessous, et dans un deuxième compartiment, un gaz comprimé choisi parmi l'air, l'azote, le gaz carbonique et leurs mélanges, et éventuellement au moins un gaz liquéfié.

Un autre objet de la présente invention consiste en l'utilisation d'au moins un copolymère à gradient de composition tel que décrit ci-dessous, dans des dispositifs aérosols à deux compartiments contenant un gaz comprimé particulier tel que décrit ci-dessous, éventuellement associé à au moins un gaz liquéfié, comme agent propulseur, pour le traitement et/ou la mise en forme de la coiffure.

D'autres caractéristiques, aspects et avantages de l'invention apparaîtront encore plus clairement à la lecture de la description et des divers exemples qui suivent.

Selon la présente invention, le dispositif aérosol à deux compartiments contient :

(a) dans un premier compartiment, une composition de traitement capillaire qui comprend, dans un milieu aqueux cosmétiquement acceptable, au moins un copolymère à gradient de composition comprenant au moins deux monomères différents et présentant un indice de polydispersité en masse (Ip) inférieur ou égal à 2,5, et
(b) dans un deuxième compartiment, un gaz comprimé choisi parmi l'air, l'azote, le gaz carbonique et leurs mélanges, l'air étant particulièrement préféré, et éventuellement au moins un gaz liquéfié.

Ledit gaz comprimé est utilisé de préférence sous une pression comprise entre 1 et 14 bars, mieux encore

comprise entre 1 et 12 bars, et encore plus préférentiellement comprise entre 9 et 11 bars.

A titre d'exemples de gaz liquéfié, on peut notamment citer les hydrocarbures comme les alcanes en $C_{1-5}$, par exemple le méthane, le propane, le butane ou le pentane, et le diméthyléther.

La quantité de gaz liquéfié est de préférence comprise entre 0 et 95 % en poids, mieux encore entre 0 et 60 % en poids par rapport au poids total de l'agent propulseur constitué des gaz comprimé et liquéfié.

L'agent propulseur particulièrement préféré, contenu dans le deuxième compartiment est constitué uniquement par au moins un gaz comprimé.

Par "copolymère à gradient de composition", on entend au sens de la présente invention un copolymère dont la distribution d'au moins un monomère des chaînes polymériques est évolutive dans un sens donné tout au long de ces chaînes et reproductible d'une chaîne à l'autre.

Les copolymères à gradient de composition utilisés dans l'invention comprennent au moins deux monomères différents, et présentent une dispersité en masse faible, ainsi que, de préférence, une dispersité en composition faible.

Une faible dispersité en masse signifie que les longueurs de chaînes sont approximativement identiques.

La dispersité en masse peut être représentée à l'aide de l'indice de polydispersité en masse (Ip) du copolymère qui est égal au rapport de la masse moléculaire moyenne en poids (Mp) à la masse moléculaire moyenne en nombre (Mn).

Le copolymère à gradient de composition utilisé dans l'invention présente un indice de polydispersité en masse inférieur ou égal à 2,5, de préférence compris entre 1,1 et 2,3, mieux encore entre 1,15 et 2,0, plus préférentiellement entre 1,2 et 1,9 ou 1,8.

La masse moléculaire moyenne en poids (Mp) du copolymère à gradient est de préférence comprise entre 5 000 et 1 000 000 g/mol, mieux encore entre 5 500 et 800 000 g/mol, et encore plus préférentiellement entre 6 000 et 500 000 g/mol.

De préférence, la masse moléculaire moyenne en nombre (Mn) du copolymère à gradient de composition est comprise entre 5 000 et 1 000 000 g/mol, mieux encore entre 5 500 et 800 000 g/mol, et encore plus préférentiellement 6 000 et 500 000 g/mol.

Les masses moléculaires moyennes en poids (Mp) et en nombre (Mn) peuvent être notamment déterminées par chromatographie liquide par perméation de gel (GPC) avec un détecteur réfractométrique et du tétrahydrofurane (THF) comme éluant, la courbe d'étalonnage étant établie avec des étalons de polystyrène linéaire.

Les copolymères à gradient de composition utilisés dans l'invention présentent également préférentiellement une faible dispersité en composition. Ceci signifie que toutes les chaînes de copolymères ont une composition (c'est-à-dire un enchaînement de monomères) approximativement analogue et sont donc homogènes en composition.

Afin de montrer que toutes les chaînes de copolymères ont une composition analogue, on pourra utiliser, de manière avantageuse, la chromatographie d'adsorption liquide (ou CAL) qui permet de séparer les chaînes de copolymères, non pas selon leur poids moléculaire mais selon leur polarité. Cette dernière permet de déterminer la composition chimique des polymères constituant le matériau, les monomères étant connus.

On pourra se reporter à la publication Macromolecules (2001), 34, 2667 qui décrit la technique de CAL.

On peut notamment définir la dispersité en composition à partir de la courbe de chromatographie d'adsorption (CAL) qui est une courbe représentant la proportion de polymères en fonction du volume d'élution. Si l'on nomme "$V^{1/2}$ min" la valeur minimum du volume d'élution à mi-hauteur de la courbe, et "$V^{1/2}$ max" la valeur maximum du volume d'élution à mi-hauteur de la courbe, la polydispersité en composition est considérée comme faible si la différence ($V^{1/2}$ max - $V^{1/2}$ min) est inférieure ou égale à 3,5, de préférence comprise entre 1 et 2,8 et mieux encore entre 1,2 et 2,5.

Par ailleurs, la courbe de CAL présente un profil de courbe de Gauss et plus particulièrement un profil de courbe de Gauss définie par la formule :

$$y = \frac{A}{w\sqrt{\frac{\pi}{2}}} \times e^{-2\frac{(x-x_0)^2}{w^2}} + y_o$$

dans laquelle :

- $x_0$ représente la valeur de x (volume d'élution) au centre du pic,
- w est égal à 2 fois l'écart type de la distribution gaussienne (soit 2σ) ou encore correspond à approximativement 0,849 fois la largeur du pic à mi-hauteur,
- A représente la surface sous le pic,
- $y_o$ représente la valeur de y correspondant à $x_0$.

La dispersité en composition peut également être définie par la valeur de w telle que définie ci dessus. De préférence, ladite valeur w est comprise entre 1 et 3, mieux encore entre 1,1 et 2,3 et encore plus préférentiellement entre 1,1 et 2,0.

Les copolymères à gradient utilisés dans l'invention peuvent être obtenus par polymérisation vivante ou pseudo-vivante.

La polymérisation vivante est une polymérisation pour laquelle la croissance des chaînes polymériques ne cesse qu'avec la disparition du monomère. La masse moyenne en nombre (Mn) croît avec la conversion. La polymérisation anionique est un exemple typique de polymérisation vivante. De telles polymérisations conduisent à des copolymères dont la dispersité en masse est faible, c'est-à-dire à des polymères d'indice de polydispersité en masse (Ip) généralement inférieur à 2.

La polymérisation pseudo-vivante est, quant à elle, associée à la polymérisation radicalaire contrôlée. Parmi les principaux types de polymérisation radicalaire contrôlée, on peut citer :

- la polymérisation radicalaire contrôlée par des nitroxydes. On peut notamment se référer aux demandes de brevet WO 96/24620 et WO 00/71501 qui décrivent les outils de cette polymérisation et leur mise en oeuvre, ainsi qu'aux articles publiés par Fischer (Chemical Reviews, 2001, 101, 3581), par Tordo et Gnanou (J. Am. Chem. Soc. 2000, 122, 5929) et Hawker (J. Am. Chem. Soc. 1999, 121, 3904);
- la polymérisation par transfert d'atome radicalaire, notamment décrite dans la demande WO 96/30421 et qui procède par l'insertion réversible sur un complexe organométallique dans une liaison de type carbone-halogène;
- la polymérisation radicalaire contrôlée par des dérivés soufrés de type xanthate, dithioesters, trithiocarbonates ou dithiocarbamates, telle que décrite dans les demandes FR 2 821 620, WO 98/01478, WO 99/35177, WO 98/58974, WO 99/31144, et dans la publication de Rizzardo & al. (Macromolecules, 1998, 31, 5559).

La polymérisation radicalaire contrôlée désigne des polymérisations pour lesquelles les réactions secondaires qui conduisent habituellement à la disparition des espèces propageantes (réaction de terminaison ou transfert) sont rendues très peu probables par rapport à la réaction de propagation grâce à un agent de contrôle des radicaux libres. Un inconvénient de ce mode de polymérisation réside dans le fait que lorsque les concentrations de radicaux libres deviennent importantes par rapport à la concentration de monomère, les réactions secondaires redeviennent déterminantes et tendent à élargir la distribution des masses.

Grâce à ces modes de polymérisation, les chaînes polymériques des copolymères à gradient de composition utilisés dans l'invention croissent simultanément et donc incorporent à chaque instant les mêmes ratios de co-monomères. Toutes les chaînes possèdent donc les mêmes structures ou des structures proches, d'où une faible dispersité en composition. Ces chaînes possèdent également un indice de polydispersité en masse faible.

Dans le cas des polymères statistiques et des polymères blocs classiques, l'évolution des monomères le long de la chaîne polymérique n'est pas graduelle et systématique.

Comme illustré par le schéma ci-après, un polymère statistique obtenu par polymérisation radicalaire classique de deux monomères se distingue d'un copolymère à gradient de composition, par la répartition des monomères qui n'est pas identique sur toutes les chaînes, et par la longueur desdites chaînes qui n'est pas identique pour toutes les chaînes.

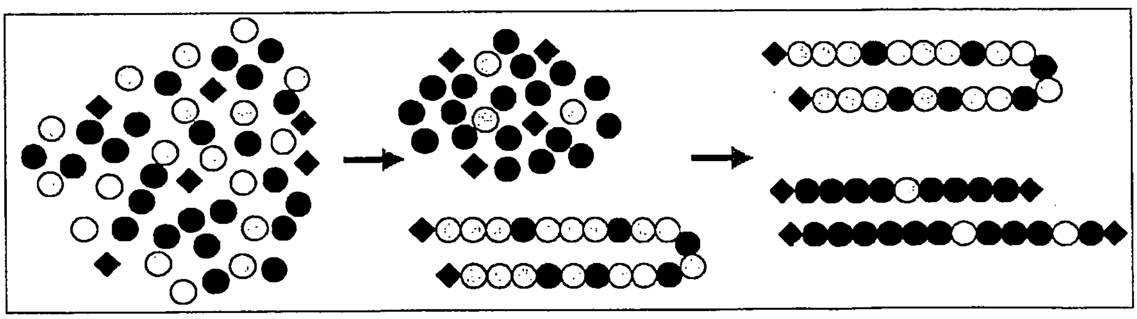

Polymérisation radicalaire conventionnelle

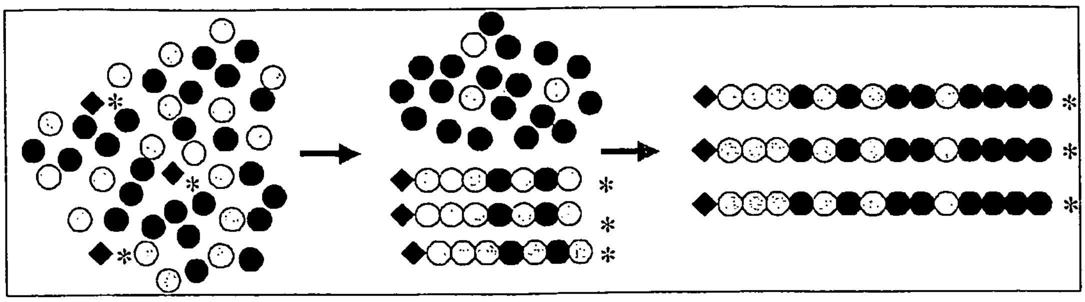

Polymérisation radicalaire contrôlée

◆ Amorceur
* Extrémité active
O Monomère le plus réactif
● Monomère le moins réactif

Pour une description théorique des copolymères à gradient de composition, il est possible de se reporter aux publications suivantes:

T. Pakula et al., Macromol. Theory Simul. 5, 987-1006 (1996) ;
A. Aksimetiev et al. J. of Chem. Physics 111, n°5 ;
M. Janco, J. Polym. Sci., Part A: Polym. Chem. (2000), 38(15), 2767-2778 ;
M. Zaremski et al., Macromolecules (2000), 33(12), 4365-4372 ;
K. Matyjaszewski & al., J. Phys. Org. Chem. (2000), 13(12), 775-786 ;
Gray, Polym. Prepr. (Am. Chem. Soc., Div. Polym. Chem.) (2001), 42(2), 337-338 ;
K. Matyjaszewski, Chem. Rev. (Washington, D. C.) (2001), 101(9), 2921-2990.

Parmi les copolymères à gradient de composition, on peut distinguer les copolymères à gradient naturel et les copolymères à gradient artificiel.

Un copolymère à gradient naturel est un copolymère à gradient de composition qui peut être obtenu par synthèse en discontinu à partir d'un mélange initial de co-monomères. La distribution dans la chaîne des divers monomères suit une loi déduite de la réactivité relative et des concentrations initiales de monomères. Ces copolymères constituent la classe la plus simple de copolymères à gradient de composition car c'est le mélange initial qui définit la propriété finale de produit.

Un copolymère à gradient artificiel est un copolymère dont on peut faire varier la concentration de monomères durant la synthèse par un artifice de procédé. Dans ce cas, on passe d'un mélange de monomères à un autre dans la chaîne du fait d'un changement subit et brusque des monomères dans le milieu réactionnel (par exemple, addition d'au moins un nouveau monomère).

La caractérisation expérimentale du gradient est apportée par la mesure en cours de polymérisation de la composition chimique du polymère. Cette mesure est faite de façon indirecte en déterminant l'évolution de la concentration des différents monomères à tout instant. Elle peut être faite par RMN et UV, par exemple.

En effet, pour les polymères préparés par polymérisation vivante ou pseudo-vivante, la longueur des chaînes est linéairement liée à la conversion. En prélevant un échantillon de la solution de polymérisation, à différents instants de la polymérisation et en mesurant la différence de teneur en chaque monomère, on accède ainsi à la composition du gradient.

Dans le polymère à gradient de composition, la distribution des compositions des chaînes est étroite. En particulier, il n'existe pas de recouvrement entre le pic chromatographique du copolymère à gradient de composition et ceux des homopolymères respectifs. Ceci signifie que le matériau obtenu en gradient est constitué de chaînes polymères de même composition alors qu'en polymérisation statistique classique, différentes sortes de chaîne coexistent, y compris celles des homopolymères respectifs.

Il est possible de caractériser les copolymères à gradient par un vecteur caractéristique de chaque copolymère.

En effet, sachant qu'il existe une infinité de polymères caractérisés par une composition chimique donnée, pour préciser un polymère il est possible de décrire la répartition des monomères le long de la chaîne. Ce qui implique une description à plusieurs variables. Ce vecteur est un point de l'espace des compositions chimiques.

Le terme exact est que G est un vecteur dont les coordonnées sont les concentrations des monomères le long de la chaîne polymérique. Ces concentrations sont définies par les règles des coefficients de réactivité de chacun des monomères, et sont donc liées à la concentration des monomères libres pendant la synthèse : du moment que le monomère n'est pas en concentration nulle dans le mélange réactionnel, il n'est pas en concentration nulle dans le polymère.
Il est donc possible de caractériser les copolymères à gradient de composition par la fonction G(x) qui définit le gradient de composition :

$$\vec{G}(x) = \sum \overrightarrow{[Mi](x)}$$

dans laquelle :

- x désigne une position normalisée sur la chaîne polymère et
- [Mi](x) est la concentration relative, en cette position x, du monomère Mi, exprimée en % en moles.

La fonction G(x) décrit donc localement la composition du copolymère à gradient.
Deux copolymères peuvent avoir une composition globale équivalente mais des répartitions locales des monomères très différentes, donc des gradients différents.
Les facteurs déterminant le gradient sont d'une part les coefficients de réactivité relatifs de chaque monomère (appelé $r_i$ pour le monomère Mi) qui dépendent principalement du type de procédé de synthèse employé (homogène, dispersé) et des solvants, et d'autre part les concentrations initiales de chacun des monomères, ainsi que les ajouts éventuels de monomères au cours de la polymérisation.
Le copolymère à gradient de composition utilisé dans l'invention comprend au moins deux monomères différents, qui peuvent être présents chacun à raison de 1 à 99% en poids par rapport au copolymère final, notamment à raison de 2-98% en poids, de préférence à raison de 5-95% en poids.
De préférence, au moins l'un des monomères du copolymère à gradient de composition est un monomère hydrophile.
Dans la présente description, on entend par "monomère hydrophile" les monomères dont les homopolymères sont solubles ou dispersibles dans l'eau, ou dont une forme ionique l'est.
Un homopolymère est dit hydrosoluble s'il forme une solution limpide lorsqu'il est en solution à 5% en poids dans l'eau, à 25°C.
Un homopolymère est dit hydrodispersible si, à 5% en poids dans l'eau, à 25°C, il forme une suspension stable de fines particules, généralement sphériques. La taille moyenne des particules constituant ladite dispersion est inférieure à 1 µm et, plus généralement, varie entre 5 et 400 nm, de préférence de 10 à 250 nm. Ces tailles de particules sont mesurées par diffusion de lumière.
De préférence, le monomère hydrophile présente une température de transition vitreuse (désignée ci-après Tg) supérieure ou égale à 20°C, mieux encore supérieure ou égale à 50°C, mais peut éventuellement avoir une Tg inférieure ou égale à 20°C.
La température de transition vitreuse (ou Tg) peut être mesurée selon la norme ASTM D3418-97, par analyse calorimétrique différentielle (ou DSC "Differential Scanning Calorimetry") avec un calorimètre sur une plage de température comprise entre -100°C et +150°C, à une vitesse de chauffe de 10°C/min dans des creusets en aluminium de 150 µl.
Parmi les monomères hydrophiles susceptibles d'être employés dans la présente invention, on peut citer les monomères suivants :

- les dérivés de (méth)acrylates d'aminoalkyle en $C_1$-$C_4$, et notamment les (méth)acrylates de N,N-di(alkyl en $C_1$-$C_4$) amino(alkyle en $C_1$-$C_6$) tels que le méthacrylate de N,N-diméthylaminoéthyle (MADAME) ou le méthacrylate de N, N-diéthylaminoéthyle (DEAMEA) ;
- les dialkylallylamines en $C_1$-$C_8$ tels que la diméthyldiallylamine ;
- la vinylamine ;
- les vinylpyridines et notamment la 2-vinylpyridine ou la 4-vinylpiridine ;

ainsi que leurs sels d'acides minéraux, ou d'acide organique, ou leurs formes quaternisées.
Parmi les acides minéraux, on peut notamment citer l'acide sulfurique, l'acide chlorhydrique, l'acide bromhydrique, l'acide iodhydrique, l'acide acétique, l'acide propionique, l'acide phosphorique ou l'acide borique.
Parmi les acides organiques, on peut citer, par exemple, les acides comportant un ou plusieurs groupes

carboxylique, sulfonique, ou phosphonique. Il peut s'agir d'acides aliphatiques linéaires, ramifiés ou cycliques, ou encore d'acides aromatiques. Ces acides peuvent comporter, en outre, un ou plusieurs hétéroatomes choisis parmi O et N, par exemple sous la forme de groupes hydroxyle.

A titre d'exemples d'acide organique, on peut citer des acides à groupe alkyle tels que l'acide acétique $CH_3COOH$, des polyacides tels que l'acide téréphtalique, et des hydroxyacides tels que l'acide citrique et l'acide tartrique.

Les agents quaternisants peuvent être des halogénures d'alkyle tel que bromure de méthyle, ou des sulfates d'alkyle tels le sulfate de méthyle ou la propane-sultone.

On peut encore citer à titre d'exemples de monomère hydrophile :

- les acides éthyléniques carboxyliques, notamment mono- ou di-carboxyliques, comportant de 3 à 20 atomes de carbone, ou leurs sels, tels que l'acide acrylique, l'acide méthacrylique, l'acide crotonique, l'acide itaconique, l'acide fumarique, l'acide maléique et l'acide vinylbenzoïque ;
- les anhydrides carboxyliques porteurs d'une double liaison vinylique, comportant de 4 à 30 atomes de carbone, tels que l'anhydride maléique ;
- les acides éthyléniques sulfoniques ou phophoniques, ou leurs sels, tels que l'acide styrène-sulfonique, l'acide acrylamidopropane-sulfonique, l'acide vinylphosphonique et leurs sels, le sel de potassium de l'acryloyloxy-3-sulfopropyle, ou le composé de formule $CH_2=CHCOOCH_2OCH_2(OH)CH_2SO_3^- Na^+$,
- l'alcool vinylique.

L'agent neutralisant peut être une base minérale, telle que LiOH, NaOH, KOH, $Ca(OH)_2$, $NH_4OH$ ; ou une base organique, par exemple, une amine primaire, secondaire ou tertiaire, notamment une alkylamine éventuellement hydroxylée comme la dibutylamine, la triéthylamine, la stéaramine, ou bien l'amino-2-méthyl-2-propanol, la monoéthanolamine, la diéthanolamine, la stéaramidopropyldiméthylamine.

On peut encore citer à titre d'exemples de monomère hydrophile :

- les amides des acides carboxyliques insaturés, comme l'acrylamide ou le méthacrylamide, et leurs amides N-substitués ou N,N-substitués tels que les N-(alkyl en $C_1$-$C_6$)(méth)acrylamides, par exemple, le N-méthylacrylamide, le N-isopropylamide, le N-butylamide et le N-tertiobutylamide, et plus particulièrement les N-(alkyl en $C_1$-$C_3$)(méth) acrylamides comme le N-méthylacrylamide ; les N,N-di(alkyl en $C_1$-$C_3$)(méth)acrylamides comme le N,N-diméthylacrylamide ; les N,N-di(alkyl en $C_1$-$C_4$)amino(alkyl en $C_1$-$C_6$)-(méth)acrylamides comme le N,N-diméthylaminopropylacrylamide (DMAPA) ou le N,N-diméthylaminopropyl-méthacrylamide (DMAPMA) ;
- les (méth)acrylates d'hydroxyalkyle notamment ceux dont le groupe alkyle comporte de 2 à 4 atomes de carbone, en particulier le (méth)acrylate d'hydroxyéthyle ;
- les (méth)acrylates de polyéthylène glycol (5 à 100 motifs oxyde d'éthylène ou OE) ou de glycol substitué ou non sur leur fonction terminale par des groupements alkyle en $C_1$-$C_4$, phosphate, phosphonate, sulfonate, par exemple, l'acrylate de glycérol, le (méth)acrylate de méthoxypolyéthylèneglycol (8 ou 12 OE); le (méth)acrylate d'hydroxypolyéthylèneglycol ;
- les (méth)acrylates d'alcoxyalkyle en $C_1$-$C_4$, tels que le (méth)acrylate de méthoxyéthyle, d'éthoxyéthyle ;
- les (méth)acrylates de polysaccharide comme l'acrylate de saccharose ;
- les vinylamides tels que le N-vinylacétamide, éventuellement cycliques comme en particulier les vinyllactames tels que la N-vinylpyrrolidone ou le N-vinylcaprolactame ;
- les vinyléthers tels que les éthers de vinyle et d'alkyle ayant 1 à 12 atomes de carbone, tels que l'éther de vinyle et de méthyle, et l'éther de vinyle et d'éthyle.

On peut encore citer comme exemples de polymère hydrophile, les composés de type bétaïne suivants :

- le méthacrylamidopropoxy-triméthylammonium ;
- le N,N-diméthyl-N-méthacryloxyéthyl-N-(3-sulfopropyl)-ammonium,
- le 3-méthacryloyléthoxycarbonylpyridinium,
- le composé de formule :

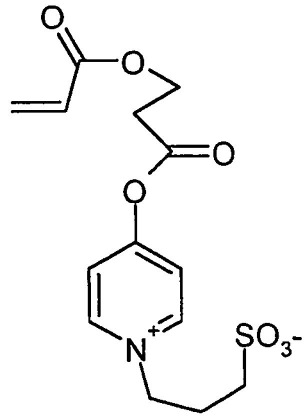

,

et

- le N-(3-sulfopropyl)-4-vinylpyridinium de formule :

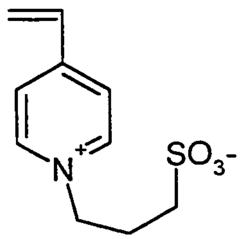

.

Au moins l'un des monomères du copolymère à gradient de composition peut également être un monomère hydrophobe, en particulier un monomère hydrophobe susceptible d'être rendu hydrophile après polymérisation, ou un mélange de tels monomères. Le(s) monomère(s) hydrophobes peuvent être rendus hydrophile(s), par exemple, par réaction chimique notamment par hydrolyse, ou par modification chimique, en particulier d'une fonction ester par incorporation de chaînes comportant un motif hydrophile, par exemple de type acide carboxylique.

De préférence, les monomères hydrophiles sont choisis parmi le méthacrylate de N,N-diméthylaminoéthyle (MADAME), l'acide acrylique, l'acide méthacrylique, l'acide crotonique, l'acide styrène-sulfonique, l'acide acrylamidopropane-sulfonique, le diméthylaminopropylméthacrylamide (DMAPMA); le styrène-sulfonate, l'acrylate d'hydroxyéthyle, l'acrylate de glycérol, le (méth)acrylate de méthoxyéthyle, le (méth)acrylate d'éthoxyéthyle, le (méth)acrylate de méthoxypolyéthylèneglycol (8 ou 12 OE); le (méth)acrylate d'hydroxypolyéthylèneglycol ; la N-vinylpyrrolidone, le N-vinylcaprolactame, l'acrylamide, le N,N-diméthylacrylamide.

Le ou les monomères hydrophiles peut ou peuvent être présent(s) à raison de 1 à 99% en poids, de préférence de 2 à 70% en poids, mieux encore de 5 à 50% en poids, encore plus préférentiellement de 10 à 30% en poids, par rapport au poids total du copolymère.

Au moins l'un des monomères du copolymère à gradient de composition utilisé dans l'invention peut être, de préférence, un monomère hydrophobe.

Parmi les monomères hydrophobes susceptibles d'être employés dans la présente invention, on peut citer :

- les hydrocarbures éthyléniques comportant de 2 à 30 atomes de carbone, tels que l'éthylène, l'isoprène et le butadiène ;
- les acrylates de formule $CH_2=CHCOOR_1$, dans laquelle $R_1$ représente un groupe hydrocarboné saturé ou non, comportant de 1 à 30 atomes de carbone, linéaire, ramifié ou cyclique, dans lequel se trouve(nt) éventuellement intercalé(s) un ou plusieurs hétéroatomes choisis parmi O, N, S et Si, ledit groupe hydrocarboné pouvant en outre être éventuellement substitué par un ou plusieurs substituants choisis parmi les groupes hydroxyle et les atomes d'halogène (Cl, Br, I et F).

A titre d'exemples de groupe hydrocarboné pour $R_1$, on peut notamment citer un groupe alkyle en $C_1$-$C_{30}$, ledit groupe alkyle pouvant être également éventuellement substitué par un ou plusieurs substituants comportant Si; un

groupe cycloalkyle en $C_3$ à $C_8$ ; un groupe aryle en $C_6$ à $C_{20}$; un groupe aralkyle en $C_7$ à $C_{30}$ (groupe alkyle en $C_1$ à $C_4$); un groupe hétérocyclique de 4 à 12 chaînons contenant un ou plusieurs hétéroatomes choisis parmi O, N et S; lesdits groupes cycloalkyle, aryle, aralkyle et hétérocyclique pouvant être également éventuellement substitués par un ou plusieurs groupes alkyle de 1 à 4 atomes de carbone, linéaires ou ramifiés, dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle et les atomes d'halogène (Cl, Br, I et F) ou Si.

On peut notamment citer comme exemples préférés de tels groupes $R_1$, les groupes méthyle, éthyle, propyle, butyle, isobutyle, tertiobutyle, hexyle, éthylhexyle, octyle, lauryle, isooctyle, isodécyle, t-butylcyclohexyle, t-butylbenzyle, furfuryle, isobornyle, éthylperfluorooctyle et propylpolydiméthylsiloxane.

$R_1$ peut aussi être un groupement $-(R_{10})_x-(OC_2H_4)_n-OR_{11}$, avec x = 0 ou 1, $R_{10}$ = groupe hydrocarboné divalent saturé ou non, tel que alkylène ou alcénylène, comportant de 1 à 30 atomes de carbone, linéaire ou ramifié, n = 5 à 100 et $R_{11}$ = H ou $CH_3$; et notamment un groupement méthoxy-$(POE)_8$-stéaryle avec POE = poly(oxyde d'éthylène).

- les méthacrylates de formule : $CH_2=C(CH_3)-COOR_2$, dans laquelle $R_2$ représente un groupe hydrocarboné saturé ou non, comportant de 1 à 30 atomes de carbone, linéaire, ramifié ou cyclique, dans lequel se trouve(nt) éventuellement intercalé(s) un ou plusieurs hétéroatomes choisis parmi O, N, S et Si, ledit groupe hydrocarboné pouvant en outre être éventuellement substitué par un ou plusieurs substituants choisis parmi les groupes hydroxyle et les atomes d'halogène (Cl, Br, I, F) ;

Comme exemple de groupes hydrocarbonés pour $R_2$, on peut notamment citer un groupe alkyle de 1 à 30 atomes de carbone, linéaire ou ramifié, ledit groupe alkyle pouvant être également éventuellement substitué par un ou plusieurs substituants comportant Si ; un groupe cycloalkyle en $C_3$ à $C_8$ ; un groupe aryle en $C_6$ à $C_{20}$ ; un groupe aralkyle en $C_7$ à $C_3$. (groupe alkyle en $C_1$ à $C_4$) ; un groupe hétérocyclique de 4 à 12 chaînons contenant un ou plusieurs hétéroatomes choisis parmi O, N, et S ; lesdits groupes cycloalkyle, aryle, aralkyle et hétérocyclique pouvant être également éventuellement substitués par un ou plusieurs groupes alkyle de 1 à 4 atomes de carbone, linéaires ou ramifiés, dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle et les atomes d'halogène (Cl, Br, I et F).

Des exemples préférés de groupes $R_2$ sont les groupes méthyle, éthyle, propyle, n-butyle, isobutyle, hexyle, éthylhexyle, octyle, lauryle, isooctyle, isodécyle, dodécyle, tertiobutylcyclohexyle, isobornyle, tertiobutylbenzyle, éthylperfluorooctyle et propylpolydiméthylsiloxane ;

$R_2$ peut aussi être un groupement $-(R_{10})_x-(OC_2H_4)_n-OR_{11}$, avec x = 0 ou 1, $R_{10}$ = groupe hydrocarboné divalent saturé ou non, tel que alkylène ou alcénylène, comportant de 1 à 30 atomes de carbone, linéaire ou ramifié, n = 5 à 100 et $R_{11}$, = H ou $CH_3$; et notamment un groupement méthoxy-$(POE)_8$-stéaryle.

Des exemples de monomères méthacrylate sont les méthacrylates de méthyle, d'éthyle, de n-butyle, d'isobutyle, de t-butylcyclohexyle, de t-butylbenzyle et d'isobornyle.

- les amides d'acides carboxyliques insaturés N- ou N,N-substitués tels que les N-(alkyl en $C_{8-30}$)(méth)acrylamides comme le N-octylacrylamide ;
- les esters de vinyle de formule : $R_3-CO-O-CH=CH_2$ où $R_3$ représente un groupe alkyle de 2 à 30 atomes de carbone, linéaire ou ramifié, notamment le propionate de vinyle, le butyrate de vinyle, l'éthylhexanoate de vinyle, le néononanoate de vinyle et le néododécanoate de vinyle ;
- les composés vinyliques de formule : $CH_2=CH-R_4$, où $R_4$ est un groupe $-OC(O)-CH_3$, un groupe cycloalkyle en $C_3$ à $C_8$ ; un groupe aryle en $C_6$ à $C_{20}$ ; un groupe aralkyle en $C_7$ à $C_{30}$ (groupe alkyle en $C_1$ à $C_4$) ; un groupe hétérocyclique de 4 à 12 chaînons contenant un ou plusieurs hétéroatomes choisis parmi O, N et S ; lesdits groupes cycloalkyle, aryle, aralkyle et hétérocyclique pouvant être éventuellement substitués par un ou plusieurs substituants choisis parmi les groupes hydroxyle, les atomes d'halogène et les groupes alkyle de 1 à 4 atomes de carbone, linéaires ou ramifiés, dans lesquels se trouve(nt) éventuellement intercalé(s) un ou plusieurs hétéroatomes choisis parmi O, N, S et P, et lesdits groupes alkyle pouvant, en outre, être éventuellement substitués par un ou plusieurs substituants choisis parmi les groupes hydroxyle et les atomes d'halogène (Cl, Br, I et F) ou Si.

Des exemples de tels monomères vinyliques sont le vinylcyclohexane, le styrène et l'acétate de vinyle.

De préférence, les monomères hydophobes sont choisis parmi :

- l'isoprène et le butadiène ;
- les acrylates de méthyle, d'éthyle, d'isobutyle, de n-butyle, de tertiobutyle, d'éthylhexyle, de furfuryle, d'isobornyle, de tertiobutylcyclohexyle, de tertiobutylbenzyle, ;

- les méthacrylates de méthyle, d'éthyle, de n-butyle, d'isobutyle, d'hexyle, d'éthylhexyle;
- les N-(alkyl en $C_{8-12}$)(méth)acrylamides tels que le N-octylacrylamide ;
- les esters de vinyle de formule : $R_3$-CO-O-CH=$CH_2$ où $R_3$ représente un groupe alkyle de 6 à 30 atomes de carbone, linéaire ou ramifié, notamment le néononanoate de vinyle et le néododécanoate de vinyle ;
- le styrène ;
- l'acétate de vinyle et le vinylcyclohexane.

Ces monomères peuvent être présents à raison de 1 à 99% en poids, de préférence de 10 à 90% en poids, mieux encore de 20 à 80% en poids, encore plus préférentiellement de 25 à 75% en poids, par rapport au poids total du copolymère.

Dans un mode de réalisation préféré, le copolymère à gradient de composition utilisé dans l'invention comprend trois monomères différents qui peuvent être présents à raison de 5-90% en poids chacun, de préférence 7-86% en poids chacun par rapport au poids total du copolymère.

En particulier, le copolymère peut comprendre 5-25% en poids d'un premier monomère, 5-25% en poids d'un second monomère et 50-90% en poids d'un troisième monomère.

Préférentiellement, le copolymère selon l'invention peut comprendre 5-25% en poids d'un monomère hydrophile, 50-90% en poids d'un monomère de Tg inférieure ou égale à 20°C et 5-25% en poids d'un monomère additionnel.

L'homme du métier saura choisir les monomères et leurs quantités en fonction du résultat recherché, en se basant sur ses connaissances générales, notamment sur la réactivité relative de chaque monomère.

Ainsi, si l'on souhaite un copolymère ayant des motifs hydrophiles dans le coeur d'une chaîne polymère, on choisira préférentiellement un amorceur di-fonctionnel et un mélange de monomères tel que la réactivité des monomères hydrophiles soit supérieure à celle des autres monomères.

Par ailleurs, on a constaté que les procédés de préparation employés permettaient d'ajuster et de moduler la ou les Tg du copolymère, et ainsi d'obtenir un copolymère à gradient de composition ayant une ou plusieurs Tg donnée(s).

Les copolymères à gradient de composition utilisés dans l'invention peuvent être préparés par l'homme du métier suivant le mode opératoire suivant :

1/ On prépare un mélange des différents monomères, éventuellement dans un solvant, de préférence dans un réacteur et sous agitation. On ajoute un amorceur de polymérisation radicalaire et un agent de contrôle de la polymérisation. Le mélange est mis de préférence sous atmosphère de gaz inerte par rapport à une polymérisation radicalaire, tel que l'azote ou l'argon.

Comme solvant éventuel de polymérisation, on peut choisir les acétates d'alkyle tels que l'acétate de butyle ou l'acétate d'éthyle, des solvants aromatiques tels que le toluène, des solvants cétoniques tels que la méthyléthylcétone, des alcools tels que l'éthanol. Dans le cas où le mélange de monomères est miscible à l'eau, celle ci peut être avantageusement utilisée comme solvant ou co-solvant.

2/ On porte le mélange sous agitation à la température de polymérisation souhaitée. Cette température est de préférence choisie dans une gamme allant de 10°C à 160°C, plus préférentiellement de 25°C à 130°C.

Le choix de la température de polymérisation est de préférence optimisé en fonction de la composition chimique du mélange de monomères. Ainsi, les monomères ayant des constantes cinétiques de propagation très élevée et une affinité pour l'agent de contrôle plus faible seront de préférence polymérisés à basse température (par exemple dans le cas d'une proportion importante de dérivés méthacryliques, on préférera une polymérisation à température comprise entre 25° et 80°C).

3/ Le milieu de polymérisation est éventuellement modifié pendant la polymérisation, avant d'atteindre 90% de conversion des monomère initiaux, par ajout supplémentaire d'un ou plusieurs monomères, notamment du mélange initial. Cet ajout peut être réalisé de différentes manières, pouvant aller de l'addition brutale en une seule fois à l'addition continue sur la durée totale de la polymérisation.

4/ La polymérisation est arrêtée lorsque le taux de conversion souhaité est atteint. De cette conversion dépend la composition globale du copolymère. De préférence, on arrête la polymérisation après avoir atteint au moins 50% de conversion, notamment au moins 60%, préférentiellement après avoir atteint au moins 90% de conversion.

5/ Les éventuels monomères résiduels peuvent être éliminés par toute méthode connue, telle que par évaporation, ou par ajout d'une quantité d'amorceur classique de polymérisation tel que les dérivés peroxydiques ou azoïques.

Dans un premier mode de réalisation, l'agent de contrôle de la polymérisation susceptible d'être employé est un nitroxyde de formule (I), seul ou en mélange :

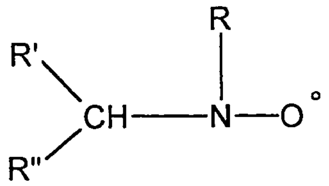 (I)

dans laquelle :

- R et R', indépendamment l'un de l'autre, sont des groupements hydrocarbonés saturés (alkyles) linéaires ou ramifiés, comportant 1 à 40 atomes de carbone, éventuellement substitués par un ou plusieurs groupements choisis parmi $-OR_4$, $-COOR_4$ et $-NHR_4$ (avec $R_4$ représentant H ou un groupement hydrocarboné saturé (alkyle) linéaire ou ramifié, comportant 1 à 40 atomes de carbone), R et R' pouvant en outre être reliés de manière à former un cycle.
  En particulier R et R' sont des groupements alkyle linéaires ou ramifiés, comportant 1 à 12 atomes de carbone, notamment des groupements méthyle, éthyle, propyle, n-butyle, iso-butyle, tert-butyle, pentyle. De préférence, R et R' sont tous les deux des groupements tert-butyle.
- R" est un groupement monovalent de masse molaire (Mp) supérieure à 16 g/mol, notamment un groupement phosphoré de formule :

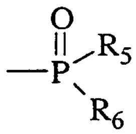

dans laquelle $R_5$ et $R_6$, indépendamment l'un de l'autre, sont des groupements hydrocarbonés saturés, de préférence alkyle, linéaires ou ramifiés, comportant 1 à 40 atomes de carbone, éventuellement substitués par un ou plusieurs groupements choisis parmi $-OR_4$, $-COOR_4$ et $-NHR_4$ (avec $R_4$ représentant H ou un groupement hydrocarboné saturé, de préférence alkyle, linéaire ou ramifié, comportant 1 à 40 atomes de carbone), $R_5$ et $R_6$ pouvant en outre être reliés de manière à former un cycle.

En particulier $R_5$ et $R_6$ sont des groupements alkyle linéaires ou ramifiés, comportant 1 à 12 atomes de carbone, notamment des groupements méthyle, éthyle, propyle, n-butyle, iso-butyle, tert-butyle, pentyle. De préférence, $R_5$ et $R_6$ sont tous les deux des groupements éthyle.

L'amorceur de polymérisation radicalaire peut être choisi parmi tous les amorceurs de polymérisation usuels, tels que les composés de type azoïque et notamment l'azobis(isobutyronitrile), ou de type peroxyde tels que les peroxydes organiques ayant 6-30 atomes de carbone, notamment le peroxyde de benzoyle.

De préférence, on respecte un ratio molaire nitroxyde/amorceur compris entre 1 et 2,5 ; ce ratio peut être compris entre 2 et 2,5 lorsque l'on considère qu'une mole d'amorceur donne naissance à deux moles de chaînes polymères, et peut être compris entre 1 et 1,25 pour des amorceurs monofonctionnels.

Dans un second mode de réalisation particulier, on peut employer comme amorceur de polymérisation radicalaire des alcoxyamines de formule (II) qui peuvent être avantageusement choisies pour initier la polymérisation et libérer en même temps le nitroxyde contrôlant cette polymérisation.

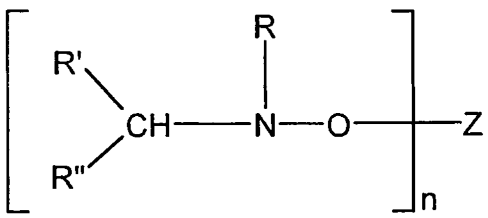 (II)

dans laquelle :

- R, R' et R" ont les significations données ci-dessus,

- n est un entier inférieur ou égal à 8, de préférence compris entre 1 et 3;
- Z est un radical monovalent ou multivalent, notamment un radical styryle, acryle ou méthacryle.

On peut également ajouter à l'alcoxyamine de formule (II), un nitroxyde de formule (I), dans une proportion allant de 0 à 20% molaires par rapport aux nombres de moles de fonctions alcoxyamines (une mole d'alcoxyamine multivalente apporte un nombre de fonctions alcoxyamine proportionnel à sa valence), de manière à améliorer la qualité du contrôle de polymérisation.

L'homme du métier saura choisir l'amorceur en fonction des besoins de l'application. Ainsi, un amorceur monofonctionnel conduira à des chaînes dissymétriques, alors qu'un amorceur polyfonctionnel conduira à des macromolécules ayant une symétrie à partir d'un coeur.

Les copolymères peuvent être présents dans la composition sous forme solubilisée, par exemple dans l'eau ou un solvant organique, ou bien sous forme de dispersion aqueuse ou organique.

Il est possible de préparer une solution aqueuse du copolymère directement par mélange du polymère avec l'eau, éventuellement en chauffant.

On peut aussi dissoudre le polymère dans un solvant organique de température d'ébullition inférieur à l'eau (par exemple l'acétone ou la méthyléthylcétone), à un taux de solide compris entre 20 et 90% en poids.

Lorsque les monomères hydrophiles sont de type acide, on peut ajouter à la solution organique, une solution de préférence d'au moins 1M de base, telle qu'un sel d'ion hydroxonium ($OH^-$), une amine (l'ammoniac), un sel de carbonate ($CO_3^{2-}$) ou d'hydrogénocarbonate ($HCO_3^-$) ou de neutralisant organique. Dans le cas de monomères hydrophiles de type amine, on peut ajouter une solution, de préférence au moins 1M, d'acide. On ajoute alors l'eau à la solution sous vive agitation dans une proportion telle que le taux de solide obtenu soit compris entre 1 et 80% en poids. Eventuellement, l'eau peut être remplacée par un mélange hydroalcoolique, dans des proportions allant de 99/1 à 50/50. On évapore le solvant en agitant la solution à 100°C. La concentration se poursuit jusqu'à l'obtention du taux de solide désiré.

Le(s) copolymère(s) à gradient de composition utilisé(s) dans le cadre de la présente invention est ou sont généralement présent(s) en une quantité allant de 0,1 à 20 % en poids, de préférence allant de 1 à 17 % en poids, mieux encore allant de 5 à 15 % en poids par rapport au poids total de la composition de traitement capillaire.

Par milieu cosmétiquement acceptable, on entend tout milieu compatible avec les matières kératiniques et notamment avec les cheveux.

Le milieu aqueux cosmétiquement acceptable comprend de l'eau et éventuellement un ou plusieurs solvants cosmétiquement acceptables. Ces solvants cosmétiquement acceptables sont notamment choisis parmi les alcools inférieurs en $C_1$-$C_4$ comme l'éthanol, l'isopropanol, le tertio-butanol, le n-butanol ; les polyols comme le propylèneglycol, et les éthers de polyols ; l'acétone ; et leurs mélanges, le solvant particulièrement préféré étant l'éthanol.

La proportion en eau peut être comprise entre 80 et 99,9 % en poids, de préférence entre 95 et 97 % en poids par rapport au poids total de la composition de traitement capillaire. De manière avantageuse, le milieu est aqueux ou un mélange hydroalcoolique. Lorsque l'alcool est présent, sa proportion dans le mélange est notamment comprise entre 1 et 99% en poids, de préférence entre 5 et 80 % en poids et encore plus préférentiellement entre 8 et 50 % en poids par rapport au poids total de la composition de traitement capillaire.

La composition de traitement capillaire selon l'invention peut contenir en outre au moins un adjuvant choisi parmi les silicones sous forme soluble, dispersée, micro-dispersée, les agents tensio-actifs non-ioniques, anioniques, cationiques et amphotères, les céramides et pseudo-céramides, les vitamines et pro-vitamines dont le panthénol, les huiles végétales, animales, minérales et synthétiques, les cires, les céramides et pseudo-céramides, les filtres solaires hydrosolubles et liposolubles, siliconés ou non siliconés, les pigments minéraux et organiques, colorés ou non colorés, les colorants, les agents nacrants et opacifiants, les agents séquestrants, les agents plastifiants, les agents solubilisants, les agents acidifiants, des agents alcalinisants, les agents épaississants minéraux et organiques, les agents anti-oxydants, les hydroxyacides, les agents de pénétration, les parfums et les agents conservateurs.

L'homme de métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 20 % en poids par rapport au poids total de la composition.

Les compositions de traitement capillaire contenues dans le dispositif selon l'invention peuvent être utilisées pour la mise en forme et/ou le maintien de la coiffure, et par exemple, en tant que compositions de fixation et/ou de maintien des cheveux, compositions de soin de cheveux, shampooings, compositions de conditionnement des cheveux, telles que des compositions destinées à apporter de la douceur aux cheveux, ou encore des compositions de maquillage des cheveux.

De préférence, le dispositif aérosol à deux compartiments est constitué par un bidon aérosol externe comportant une poche interne soudée hermétiquement à une valve. La composition est introduite dans la poche interne et un gaz comprimé est introduit entre la poche et le bidon à une pression suffisante pour faire sortir le produit sous forme d'un spray à travers l'orifice d'une buse. Un tel dispositif est commercialisé sous le nom EP SPRAY par la société EP-SPRAY

SYSTEM SA.

Plus particulièrement, la présente invention concerne aussi l'utilisation du produit vaporisé par le dispositif aérosol selon l'invention, pour la mise en forme et/ou le maintien de la coiffure, par exemple, comme laque pour cheveux.

La présente invention concerne également un procédé de coiffage, comprenant l'étape consistant à vaporiser la composition de traitement capillaire contenue dans le dispositif aérosol selon l'invention, sur les cheveux mouillés ou non.

Les exemples suivants sont donnés à titre illustratif de la présente invention.

EXEMPLES

Exemple 1

La composition suivante a été préparée en mélangeant les ingrédients indiqués ci-dessous.

| | Quantité (% en poids) |
|---|---|
| Copolymère à gradient : Poly(acide méthacrylique/styrène)/poly(acrylate de butyle) | 5 |
| Eau | 95 |

On a introduit la composition préparée ci-dessus dans le dispositif de distribution aérosol commercialisé sous le nom EP SPRAY par la société EP SPRAY SYSTEM SA décrit ci-dessus. Une valve de référence 6001 format D6 est fixée sur un bidon aérosol classique et le diffuseur est un diffuseur à buse tourbillonnaire.

La poche est remplie avec la composition comme indiqué ci-dessus. De l'air comprimé est introduit entre la poche et le bidon.

L'application de ce produit sur des cheveux secs ou humides permet d'obtenir une bonne fixation sans blanchiment des cheveux.

Exemple 2

| | Quantité (% en poids) |
|---|---|
| Copolymère à gradient : Poly(acide méthacrylique/styrène)/poly(acrylate d'éthyle) | 5 |
| Eau | 95 |

On a opéré comme dans l'exemple 1.

L'application de ce produit sur des cheveux secs ou humides permet d'obtenir une bonne fixation sans blanchiment des cheveux.

Exemple 3

| | Quantité (% en poids) |
|---|---|
| Copolymère à gradient : Poly(acide méthacrylique/styrène)/poly(acrylate de méthyle/ acrylate de butyle) | 5 |
| Eau | 95 |

On a opéré comme dans l'exemple 1.

L'application de ce produit sur des cheveux secs ou humides permet d'obtenir une bonne fixation sans blanchiment des cheveux.

## Revendications

**1.** Dispositif aérosol à deux compartiments comprenant:

(a) dans un premier compartiment, une composition de traitement capillaire qui comprend, dans un milieu aqueux cosmétiquement acceptable, au moins un copolymère à gradient de composition comprenant au moins deux monomères différents et présentant un indice de polydispersité en masse (Ip) inférieur ou égal à 2,5, et
(b) dans un deuxième compartiment, un gaz comprimé choisi parmi l'air, l'azote, le gaz carbonique et leurs mélanges, et éventuellement au moins un gaz liquéfié.

**2.** Dispositif aérosol selon la revendication 1, **caractérisé en ce que** le gaz comprimé est l'air.

**3.** Dispositif aérosol selon la revendication 1 ou 2, **caractérisé en ce que** la pression du gaz comprimé est comprise entre 1 et 14 bars.

**4.** Dispositif aérosol selon la revendication 3, **caractérisé en ce que** la pression du gaz comprimé est comprise entre 9 et 11 bars.

**5.** Dispositif aérosol selon l'une quelconque des revendications précédentes, **caractérisé en ce que** le gaz liquéfié est choisi parmi les hydrocarbures et le diméthyléther.

**6.** Dispositif aérosol selon la revendication 5, **caractérisé en ce que** les hydrocarbures sont choisis parmi les alcanes en $C_{1-5}$.

**7.** Dispositif aérosol selon l'une quelconque des revendications précédentes, **caractérisé en ce que** le gaz liquéfié est contenu en une quantité allant de 0 à 95 % en poids, de préférence entre 0 et 60 % en poids par rapport au poids total de l'agent propulseur constitué des gaz comprimé et liquéfié.

**8.** Dispositif aérosol selon l'une quelconque des revendications précédentes, **caractérisé en ce que** l'indice de polydispersité en masse (Ip) est compris entre 1,1 et 2,3.

**9.** Dispositif aérosol selon l'une quelconque des revendications précédentes, **caractérisé en ce que** la masse moléculaire moyenne en poids du copolymère à gradient de composition est comprise entre 5 000 et 1 000 000 g/mol.

**10.** Dispositif aérosol selon l'une quelconque des revendications précédentes, **caractérisé en ce que** la masse moléculaire moyenne en nombre du copolymère à gradient de composition est comprise entre 5 000 et 1 000 000 g/mol.

**11.** Dispositif aérosol selon l'une quelconque des revendications précédentes, **caractérisé en ce que** le copolymère à gradient de composition est tel que sur la courbe de chromatographie d'adsorption (CAL) représentant la proportion de polymères en fonction du volume d'élution, la différence ($V^{1/2}$ max - $V^{1/2}$ min) est inférieure ou égale à 3,5, de préférence comprise entre 1 et 2,8, "$V^{1/2}$ min" étant la valeur minimum du volume d'élution à mi-hauteur de la courbe, et "$V^{1/2}$ max" étant la valeur maximum du volume d'élution à mi-hauteur de la courbe.

**12.** Dispositif aérosol selon l'une quelconque des revendications précédentes, **caractérisé en ce qu'**au moins l'un des monomères du copolymère à gradient de composition est un monomère hydrophile.

**13.** Dispositif aérosol selon la revendication 12, **caractérisé en ce que** le monomère hydrophile est choisi parmi :

- les dérivés de (méth)acrylates d'aminoalkyle en $C_1$-$C_4$ ;
- les dialkylallylamines en $C_1$-$C_8$ ;
- la vinylamine ;
- les vinylpyridines ;

ainsi que leurs sels d'acides minéraux, ou d'acide organique, ou leurs formes quaternisées ;

- les acides éthyléniques carboxyliques comportant de 3 à 20 atomes de carbone, ou leurs sels ;
- les anhydrides carboxyliques porteurs d'une double liaison vinylique, comportant de 4 à 30 atomes de carbone ;
- les acides éthyléniques sulfoniques ou phosphoniques, et leurs sels ;
- l'alcool vinylique ;
- l'acrylamide ou le méthacrylamide, les N-(alkyl en $C_1$-$C_6$)(méth)acrylamides, les N,N-di(alkyl en $C_1$-$C_3$)(méth) acrylamides, les N,N-di(alkyl en $C_1$-$C_4$)amino(alkyl en $C_1$-$C_6$)(méth)acrylamides
- les (méth)acrylates d'hydroxy(alkyle en $C_2$-$C_4$) ;
- les (méth)acrylates de polyéthylèneglycol (5 à 100 motifs oxyde d'éthylène) ou de glycol substitué ou non sur leur fonction terminale par des groupements alkyle en $C_{1-4}$, phosphate, phosphonate, sulfonate;
- les (méth)acrylates d'alcoxyalkyle en $C_{1-4}$ ;
- les (méth)acrylates de polysaccharide ;
- les vinylamides éventuellement cycliques ;
- les vinyléthers ;
- le méthacrylamidopropoxy-triméthylammonium ;
- le N,N-diméthyl-N-méthacryloxyéthyl-N-(3-sulfopropyl)ammonium,
- le 3-méthacryloyléthoxycarbonylpyridinium,
- le composé de formule :

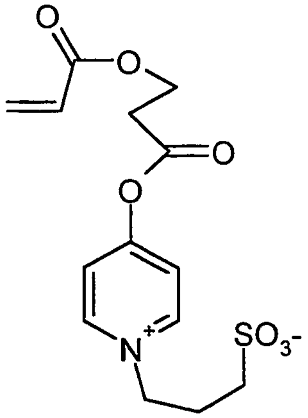

,

et

- le N-(3-sulfopropyl)-4-vinylpyridinium de formule :

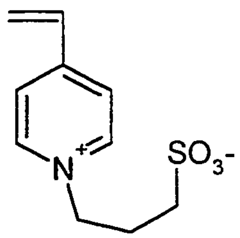

.

**14.** Dispositif aérosol selon la revendication 13, **caractérisé en ce que** le monomère hydrophile est choisi parmi le méthacrylate de N,N-diméthylaminoéthyle (MADAME), l'acide acrylique, l'acide méthacrylique, l'acide crotonique, l'acide styrène-sulfonique, l'acide acrylamidopropane-sulfonique, le diméthylaminopropylméthacrylamide (DMAPMA); le styrène-sulfonate, l'acrylate d'hydroxyéthyle, l'acrylate de glycérol, le (méth)acrylate de méthoxyéthyle, le (méth)acrylate d'éthoxyéthyle, le (méth)acrylate de méthoxypolyéthylèneglycol (8 ou 12 OE); le (méth)acrylate d'hydroxypolyéthylèneglycol ; la N-vinylpyrrolidone, le N-vinylcaprolactame, l'acrylamide et le N,N-diméthylacrylamide.

**15.** Dispositif aérosol selon l'une quelconque des revendications précédentes, **caractérisé en ce qu'**au moins l'un des

monomères du copolymère à gradient de composition est un monomère hydrophobe.

**16.** Dispositif aérosol selon la revendication 15, **caractérisé en ce que** le monomère hydrophobe est choisi parmi :

- les hydrocarbures éthyléniques comportant de 2 à 30 atomes de carbone ;
- les acrylates de formule $CH_2$=CHCOO$R_1$, dans laquelle $R_1$ représente un groupe hydrocarboné saturé ou non, comportant de 1 à 30 atomes de carbone, linéaire, ramifié ou cyclique, dans lequel se trouve(nt) éventuellement intercalé(s) un ou plusieurs hétéroatomes choisis parmi O, N, S et Si, ledit groupe hydrocarboné étant en outre éventuellement substitué par un ou plusieurs substituants choisis parmi les groupes hydroxyle et les atomes d'halogène,

ou $R_1$ représente un groupement $-(R_{10})_x-(OC_2H_4)_n-OR_{11}$, avec x = 0 ou 1, $R_{10}$ = groupe hydrocarboné divalent saturé ou non, comportant de 1 à 30 atomes de carbone, linéaire ou ramifié, n = 5 à 100 et $R_{11}$ = H ou $CH_3$ ;

- les méthacrylates de formule : $CH_2=C(CH_3)-COOR_2$, dans laquelle $R_2$ représente un groupe hydrocarboné saturé ou non, comportant de 1 à 30 atomes de carbone, linéaire, ramifié ou cyclique, dans lequel se trouve (nt) éventuellement intercalé(s) un ou plusieurs hétéroatomes choisis parmi O, N, S et Si, ledit groupe hydrocarboné étant en outre éventuellement substitué par un ou plusieurs substituants choisis parmi les groupes hydroxyle et les atomes d'halogène,

ou $R_2$ représente $-(R_{10})_x-(OC_2H_4)_n-OR_{11}$, avec x = 0 ou 1, $R_{10}$ = groupe hydrocarboné divalent saturé ou non, comportant de 1 à 30 atomes de carbone, linéaire ou ramifié, n = 5 à 100 et $R_{11}$ = H ou $CH_3$ ;

- les N-(alkyl en $C_{8-30}$)(méth)acrylamides ;
- les esters de vinyle de formule : $R_3$-CO-O-CH=$CH_2$ où $R_3$ représente un groupe alkyle de 2 à 12 atomes de carbone, linéaire ou ramifié ;
- les composés vinyliques de formule : $CH_2=CH-R_4$, où $R_4$ est un groupe -OC(O)-$CH_3$, un groupe cycloalkyle en $C_3$ à $C_8$ ; un groupe aryle en $C_6$ à $C_{20}$ ; un groupe aralkyle en $C_7$ à $C_{30}$ (groupe alkyle en $C_1$ à $C_4$) ; un groupe hétérocyclique de 4 à 12 chaînons contenant un ou plusieurs hétéroatomes choisis parmi O, N, et S ; lesdits groupes cycloalkyle, aryle, aralkyle et hétérocyclique étant éventuellement substitués par un ou plusieurs substituants choisis parmi les groupes hydroxyle, les atomes d'halogène et les groupes alkyles de 1 à 4 atomes de carbone, linéaires ou ramifiés, dans lesquels se trouve(nt) éventuellement intercalé(s) un ou plusieurs hétéroatomes choisis parmi 0, N, S et P, et lesdits groupes alkyle étant, en outre, éventuellement substitués par un ou plusieurs substituants choisis parmi les groupes hydroxyle et les atomes d' halogène ou Si.

**17.** Dispositif aérosol selon la revendication 16, **caractérisé en ce que** le monomère hydrophobe est choisi parmi :

- l'isoprène et le butadiène ;
- les acrylates de méthyle, d'éthyle, d'isobutyle, de n-butyle, de tertiobutyle, d'éthylhexyle, de furfuryle, d'isobornyle, de tertiobutylcyclohexyle, de tertiobutylbenzyle ;
- les méthacrylates de méthyle, d'éthyle, de n-butyle, d'isobutyle, d'hexyle, d'éthylhexyle;
- les N-(alkyl en $C_{6-12}$)(méth)acrylamides ;
- les esters de vinyle de formule : $R_3$-CO-O-CH=$CH_2$ où $R_3$ représente un groupe alkyle de 6 à 12 atomes de carbone, linéaire ou ramifié;
- le styrène,
- l'acétate de vinyle et le vinylcyclohexane.

**18.** Dispositif aérosol selon l'une quelconque des revendications précédentes, **caractérisé en ce que** le(s) copolymère (s) à gradient de composition est ou sont présent(s) en une quantité allant de 0,1 à 20 % en poids, de préférence de 1 à 17 % en poids par rapport au poids total de la composition de traitement capillaire.

**19.** Dispositif aérosol selon l'une quelconque des revendications précédentes, **caractérisé en ce que** le copolymère à gradient de composition est présent sous forme solubilisée, ou bien sous forme de dispersion aqueuse ou organique.

**20.** Dispositif aérosol selon l'une quelconque des revendications précédentes, **caractérisé en ce que** le milieu aqueux cosmétiquement acceptable comprend de l'eau et éventuellement un ou plusieurs solvants cosmétiquement acceptables.

**21.** Dispositif aérosol selon la revendication 20, **caractérisé en ce que** le (s) solvant(s) cosmétiquement acceptable (s) est ou sont choisi(s) parmi les alcools inférieurs en $C_1$-$C_4$, les polyols, les éthers de polyols, l'acétone et leurs mélanges.

**22.** Dispositif aérosol selon l'une quelconque des revendications précédentes, **caractérisé en ce que** l'eau est présente en une quantité comprise entre 80 et 99,9 %, de préférence entre 95 et 97 % en poids par rapport au poids total de la composition de traitement capillaire.

**23.** Dispositif aérosol selon l'une quelconque des revendications précédentes, **caractérisé en ce que** la composition de traitement capillaire comprend en outre au moins un adjuvant choisi parmi les silicones sous forme soluble, dispersée, micro-dispersée, les agents tensio-actifs non-ioniques, anioniques, cationiques et amphotères, les céramides et pseudo-céramides, les vitamines et pro-vitamines dont le panthénol, les huiles végétales, animales, minérales et synthétiques, les cires, les céramides et pseudo-céramides, les filtres solaires hydrosolubles et liposolubles, siliconés ou non siliconés, les pigments minéraux et organiques, colorés ou non colorés, les colorants, les agents nacrants et opacifiants, les agents séquestrants, les agents plastifiants, les agents solubilisants, les agents acidifiants, des agents alcalinisants, les agents épaississants minéraux et organiques, les agents anti-oxydants, les hydroxyacides, les agents de pénétration, les parfums et les agents conservateurs.

**24.** Utilisation du produit vaporisé par le dispositif aérosol selon l'une quelconque des revendications 1 à 23, pour la mise en forme et/ou le maintien de la coiffure.

**25.** Procédé de coiffage comprenant l'étape qui consiste à vaporiser la composition de traitement capillaire contenue dans le dispositif aérosol selon l'une quelconque des revendications 1 à 23, sur les cheveux mouillés ou non.

**26.** Utilisation d'au moins un copolymère à gradient de composition comprenant au moins deux monomères différents et présentant un indice de polydispersité en masse (Ip) inférieur ou égal à 2,5, dans un dispositif aérosol à deux compartiments contenant un gaz comprimé choisi parmi l'air, l'azote, le gaz carbonique et leurs mélanges, et éventuellement un gaz liquéfié, comme agent propulseur, pour le traitement et/ou la mise en forme de la coiffure.

**27.** Utilisation selon la revendication 26, **caractérisée en ce que** le gaz comprimé est l'air.

**28.** Utilisation selon la revendication 26 ou 27, **caractérisée en ce que** la pression du gaz comprimé est comprise entre 1 et 14 bars.

**29.** Utilisation selon la revendication 28, **caractérisée en ce que** la pression du gaz comprimé est comprise entre 9 et 11 bars.

**30.** Utilisation selon l'une quelconque des revendications 26 à 29, **caractérisée en ce que** le gaz liquéfié est choisi parmi les hydrocarbures et le diméthyléther.

**31.** Utilisation selon la revendication 30, **caractérisée en ce que** les hydrocarbures sont choisis parmi les alcanes en $C_{1-5}$.

**32.** Utilisation selon l'une quelconque des revendications précédentes, **caractérisée en ce que** le gaz liquéfié est contenu en une quantité allant de 0 à 95 % en poids, de préférence entre 0 et 60 % en poids par rapport au poids total de l'agent propulseur constitué des gaz comprimé et liquéfié.

**33.** Utilisation selon l'une quelconque des revendications 26 à 32, **caractérisée en ce que** l'indice de polydispersité en masse (Ip) est compris entre 1,1 et 2,3.

**34.** Utilisation selon l'une quelconque des revendications 26 à 33, **caractérisée en ce que** la masse moléculaire moyenne en poids du copolymère à gradient de composition est comprise entre 5 000 et 1 000 000 g/mol.

**35.** Utilisation selon l'une quelconque des revendications 26 à 34, **caractérisée en ce que** la masse moléculaire moyenne en nombre du copolymère à gradient de composition est comprise entre 5 000 et 1 000 000 g/mol.

**36.** Utilisation selon l'une quelconque des revendications 26 à 35, **caractérisée en ce que** le copolymère à gradient de composition est tel que sur la courbe de chromatographie d'adsorption (LAC), représentant la proportion de

polymères en fonction du volume d'élution, la différence ($V^{1/2}$ max - $V^{1/2}$ min) est inférieure ou égale à 3,5, de préférence comprise entre 1 et 2,8, "$V^{1/2}$ min" étant la valeur minimum du volume d'élution à mi-hauteur de la courbe, et "$V^{1/2}$ max" étant la valeur maximum du volume d'élution à mi-hauteur de la courbe.

**37.** Utilisation selon l'une quelconque des revendications 26 à 36, **caractérisée en ce qu'**au moins l'un des monomères du copolymère à gradient de composition est un monomère hydrophile.

**38.** Utilisation selon la revendication 37, **caractérisée en ce que** le monomère hydrophile choisi parmi :

- les dérivés de (méth)acrylates d'aminoalkyle en $C_1$-$C_4$ ;
- les dialkylallylamines en $C_1$-$C_8$ ;
- la vinylamine ;
- les vinylpyridines ;

ainsi que leurs sels d'acides minéraux, ou d'acide organique, ou leurs formes quaternisées ;

- les acides éthyléniques carboxyliques comportant de 3 à 20 atomes de carbone, ou leurs sels ;
- les anhydrides carboxyliques porteurs d'une double liaison vinylique, comportant de 4 à 30 atomes de carbone ;
- les acides éthyléniques sulfoniques ou phosphoniques, et leurs sels ;
- l'alcool vinylique ;
- l'acrylamide ou le méthacrylamide, les N-(alkyl en $C_1$-$C_6$)(méth)acrylamides, les N,N-di(alkyl en $C_1$-$C_3$)(méth) acrylamides, les N,N-di(alkyl en $C_1$-$C_4$)amino(alkyl en $C_1$-$C_6$)(méth)acrylamides ;
- les (méth)acrylates d'hydroxy(alkyle en $C_2$-$C_4$) ;
- les (méth)acrylates de polyéthylèneglycol (5 à 100 motifs oxyde d'éthylène) ou de glycol substitué ou non sur leur fonction terminale par des groupements alkyle en $C_1$-$C_4$, phosphate, phosphonate, sulfonate ;
- les (méth)acrylates d'alcoxyalkyle en en $C_1$-$C_4$ ;
- les (méth)acrylates de polysaccharide ;
- les vinylamides éventuellement cycliques ;
- les vinyléthers ;
- le méthacrylamidopropoxy-triméthylammonium ;
- le N,N-diméthyl-N-méthacryloxyéthyl-N-(3-sulfopropyl)ammonium,
- le 3-méthacryloyléthoxycarbonylpyridinium,
- le composé de formule :

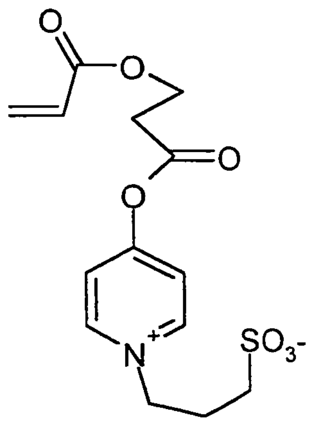

,

et
- le N-(3-sulfopropyl)-4-vinylpyridinium de formule :

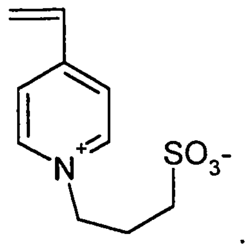

**39.** Utilisation selon la revendication 38, **caractérisée en ce que** le monomère hydrophile est choisi parmi le méthacrylate de N,N-diméthylaminoéthyle (MADAME), l'acide acrylique, l'acide méthacrylique, l'acide crotonique, l'acide styrène-sulfonique, l'acide acrylamidopropane-sulfonique, le diméthylaminopropylméthacrylamide (DMAPMA); le styrène-sulfonate, l'acrylate d'hydroxyéthyle, l'acrylate de glycérol, le (méth)acrylate de méthoxyéthyle, le (méth)acrylate d'éthoxyéthyle, le (méth)acrylate de méthoxypolyéthylèneglycol (8 ou 12 OE); le (méth)acrylate d'hydroxypolyéthylèneglycol ; la N-vinylpyrrolidone, le N-vinylcaprolactame, l'acrylamide et le N,N-diméthylacrylamide.

**40.** Utilisation selon l'une quelconque des revendications 26 à 39, **caractérisée en ce qu'**au moins l'un des monomères du copolymère à gradient de composition est un monomère hydrophobe.

**41.** Utilisation selon la revendication 40, **caractérisée en ce que** le monomère hydrophobe est choisi parmi :

- les hydrocarbures éthyléniques comportant de 2 à 30 atomes de carbone ;
- les acrylates de formule $CH_2$=$CHCOOR_1$, dans laquelle $R_1$ représente un groupe hydrocarboné saturé ou non, comportant de 1 à 30 atomes de carbone, linéaire, ramifié ou cyclique, dans lequel se trouve(nt) éventuellement intercalé(s) un ou plusieurs hétéroatomes choisis parmi O, N, S et Si, ledit groupe hydrocarboné étant en outre éventuellement substitué par un ou plusieurs substituants choisis parmi les groupes hydroxyle et les atomes d'halogène,

ou $R_1$ représente un groupement $-(R_{10})_x-(OC_2H_4)_n-OR_{11}$, avec x = 0 ou 1, $R_{10}$ = groupe hydrocarboné divalent saturé ou non, comportant de 1 à 30 atomes de carbone, linéaire ou ramifié, n = 5 à 100 et $R_{11}$ = H ou $CH_3$ ;

- les méthacrylates de formule : $CH_2$=$C(CH_3)$-$COOR_2$, dans laquelle $R_2$ représente un groupe hydrocarboné saturé ou non, comportant de 1 à 30 atomes de carbone, linéaire, ramifié ou cyclique, dans lequel se trouve (nt) éventuellement intercalé(s) un ou plusieurs hétéroatomes choisis parmi O, N, S et Si, ledit groupe hydrocarboné étant en outre éventuellement substitué par un ou plusieurs substituants choisis parmi les groupes hydroxyle et les atomes d'halogène,
ou $R_2$ représente $-(R_{10})_x-(OC_2H_4)_n-OR_{11}$, avec x = 0 ou 1, $R_{10}$ = groupe hydrocarboné divalent saturé ou non, comportant de 1 à 30 atomes de carbone, linéaire ou ramifié, n = 5 à 100 et $R_{11}$ = H ou $CH_3$ ;

  - - les N-(alkyl en $C_{8-30}$)(méth)acrylamides ;

- les esters de vinyle de formule : $R_3$-CO-O-CH=$CH_2$ où $R_3$ représente un groupe alkyle de 2 à 12 atomes de carbone, linéaire ou ramifié ;
- les composés vinyliques de formule : $CH_2$=CH-$R_4$, où $R_4$ est un groupe -OC(O)-$CH_3$, un groupe cycloalkyle en $C_3$ à $C_8$ ; un groupe aryle en $C_6$ à $C_{20}$ ; un groupe aralkyle en $C_7$ à $C_{30}$ (groupe alkyle en $C_1$ à $C_4$) ; un groupe hétérocyclique de 4 à 12 chaînons contenant un ou plusieurs héréatomes choisis parmi O, N, et S ; lesdits groupes cycloalkyle, aryle, aralkyle et hétérocyclique étant éventuellement substitués par un ou plusieurs substituants choisis parmi les groupes hydroxyle, les atomes d'halogène, et les groupes alkyle de 1 à 4 atomes de carbone, linéaires ou ramifiés, dans lesquels se trouve(nt) éventuellement intercalé(s) un ou plusieurs hétéroatomes choisis parmi O, N, S et P, et lesdits groupes alkyle étant, en outre, éventuellement substitués par un ou plusieurs substituants choisis parmi les groupes hydroxyle et les atomes d'halogène ou Si.

**42.** Utilisation selon la revendication 41, **caractérisée en ce que** le monomère hydrophobe est choisi parmi :

- l'isoprène et le butadiène ;
- les acrylates de méthyle, d'éthyle, d'isobutyle, de n-butyle, de tertiobutyle, d'éthylhexyle, de furfuryle, d'isobornyle, de tertiobutylcyclohexyle, de tertiobutylbenzyle ;
- les méthacrylates de méthyle, d'éthyle, de n-butyle, d'isobutyle, d'hexyle, d'éthylhexyle;
- les N-(alkyl en $C_{6-12}$)(méth)acrylamides ;
- les esters de vinyle de formule : $R_3$-CO-O-CH=$CH_2$ où $R_3$ représente un groupe alkyle de 6 à 12 atomes de carbone, linéaire ou ramifié ;
- le styrène ;
- l'acétate de vinyle et le vinylcyclohexane

**43.** Utilisation selon l'une quelconque des revendications 26 à 42, **caractérisée en ce que** le copolymère à gradient de composition est présent sous forme solubilisée, ou bien sous forme de dispersion aqueuse ou organique.

**Claims**

**1.** Two-compartment aerosol device comprising:

(a) in a first compartment, a hair treatment composition which comprises, in a cosmetically acceptable aqueous medium, at least one compositionally gradient copolymer comprising at least two different monomers and exhibiting a weight polydispersity index (PI) of less than or equal to 2.5, and
(b) in a second compartment, a compressed gas chosen from air, nitrogen, carbon dioxide and their mixtures, and optionally at least one liquefied gas.

**2.** Aerosol device according to Claim 1, **characterized in that** the compressed gas is air.

**3.** Aerosol device according to Claim 1 or 2, **characterized in that** the pressure of the compressed gas is between 1 and 14 bar.

**4.** Aerosol device according to Claim 3, **characterized in that** the pressure of the compressed gas is between 9 and 11 bar.

**5.** Aerosol device according to any one of the preceding claims, **characterized in that** the liquefied gas is chosen from hydrocarbons and dimethyl ether.

**6.** Aerosol device according to Claim 5, **characterized in that** the hydrocarbons are chosen from $C_{1-5}$ alkanes.

**7.** Aerosol device according to any one of the preceding claims, **characterized in that** the liquefied gas is present in an amount ranging from 0 to 95% by weight, preferably between 0 and 60o by weight, with respect to the total weight of the propellant composed of the compressed and liquefied gases.

**8.** Aerosol device according to any one of the preceding claims, **characterized in that** the weight polydispersity index (PI) is between 1.1 and 2.3.

**9.** Aerosol device according to any one of the preceding claims, **characterized in that** the weight-average molecular weight of the compositionally gradient copolymer is between 5000 and 1 000 000 g/mol.

**10.** Aerosol device according to any one of the preceding claims, **characterized in that** the number-average molecular weight of the compositionally gradient copolymer is between 5000 and 1 000 000 g/mol.

**11.** Aerosol device according to any one of the preceding claims, **characterized in that** the compositionally gradient copolymer is such that, on the adsorption chromatography (LAC) curve representing the proportion of polymers as a function of the elution volume, the difference ($V^{1/2}$ max - $V^{1/2}$ min) is less than or equal to 3.5, preferably between 1 and 2.8, "$V^{1/2}$ min" being the minimum value of the elution volume at mid-height of the curve and "$V^{1/2}$ max" being the maximum value of the elution volume at mid-height of the curve.

**12.** Aerosol device according to any one of the preceding claims, **characterized in that** at least one of the monomers of the compositionally gradient copolymer is a hydrophilic monomer.

**13.** Aerosol device according to claim 12, **characterized in that** the hydrophilic monomer is chosen from:

- amino($C_1$-$C_4$ alkyl) (meth)acrylate derivatives;
- di ($C_1$-$C_8$ alkyl)allylamines;
- vinylamine;
- vinylpyridines;

and their salts with inorganic acids or with organic acids, or their quaternized forms;

- ethylenic carboxylic acids comprising from 3 to 20 carbon atoms, or their salts;
- carboxylic anhydrides carrying a vinyl double bond and comprising from 4 to 30 carbon atoms;
- sulfonic or phosphonic ethylenic acids, and their salts;
- vinyl alcohol;
- acrylamide or methacrylamide, N-($C_1$-$C_6$ alkyl) (meth)-acrylamides, N,N-di($C_1$-$C_3$ alkyl) (meth)acrylamides or N,N-di($C_1$-$C_4$alkyl) amino ($C_1$-$C_6$ alkyl) (meth) acrylamides;
- hydroxy($C_2$-$C_9$alkyl) (meth)acrylates;
- polyethylene glycol (5 to 100 ethylene oxide units) or glycol (meth)acrylates which are or are not substituted on their terminal functional group by $C_{1-4}$ alkyl, phosphate, phosphonate or sulfonate groups;
- $C_{1-4}$ alkoxyalkyl (meth)acrylates;
- polysaccharide (meth)acrylates;
- optionally cyclic vinylamides;
- vinyl ethers;
- methacrylamidopropoxytrimethylammonium;
- N,N-dimethyl-N-methacryloyloxyethyl-N-(3-sulfopropyl)-ammonium;
- 3-methacryloylethoxycarbonylpyridinium;
- the compound of formula:

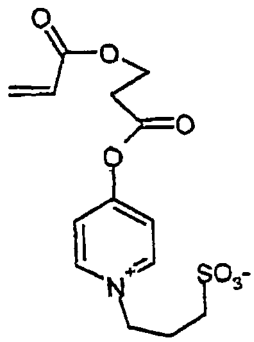

,

and
- N-(3-sulfopropyl)-4-vinylpyridinium of formula:

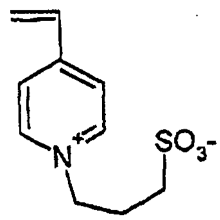

.

**14.** Aerosol device according to Claim 13, **characterized in that** the hydrophilic monomer is chosen from N,N-dimethylaminoethyl methacrylate (MADAME), acrylic acid, methacrylic acid, crotonic acid, styrenesulfonic acid, acrylamidopropanesulfonic acid, dimethylaminopropylmethacrylamide (DMAPMA), styrenesulfonate, hydroxyethyl acrylate, glyceryl acrylate, methoxyethyl (meth)acrylate, ethoxyethyl (meth)acrylate, methoxypolyethylene glycol (8 or 12 EO) (meth)acrylate, hydroxypolyethylene glycol (meth)acrylate, N-vinylpyrrolidone, N-vinylcaprolactam, acrylamide and N,N-dimethylacrylamide.

**15.** Aerosol device according to any one of the preceding claims, **characterized in that** at least one of the monomers of the compositionally gradient copolymer is a hydrophobic monomer.

**16.** Aerosol device according to Claim 15, **characterized in that** the hydrophobic monomer is chosen from:

- ethylenic hydrocarbons comprising from 2 to 30 carbon atoms;
- acrylates of formula $CH_2$=CHCOOR$_1$, in which $R_1$ represents a saturated or unsaturated, linear, branched or cyclic, hydrocarbon group comprising from 1 to 30 carbon atoms in which one or more heteroatoms chosen from O, N, S and Si is/are optionally inserted, said hydrocarbon group additionally being optionally substituted by one or more substituents chosen from hydroxyl groups and halogen atoms,

or $R_1$ represents a $-(R_{10})_x-(OC2H9)_n-OR_{11}$ group, with x = 0 or 1, $R_{10}$ = saturated or unsaturated, linear or branched, divalent hydrocarbon group comprising from 1 to 30 carbon atoms, n = 5 to 100 and $R_{11}$ = H or $CH_3$;

- methacrylates of formula $CH_2=C(CH_3)-COOR_2$, in which $R_2$ represents a saturated or unsaturated, linear, branched or cyclic, hydrocarbon group comprising from 1 to 30 carbon atoms in which one or more heteroatoms chosen from O, N, S and Si is/are optionally inserted, said hydrocarbon group additionally being optionally substituted by one or more substituents chosen from hydroxyl groups and halogen atoms,

or $R_2$ represents $-(R_{10})_x-(OC_2H_4)_n-OR_{11}$, with x = 0 or 1, $R_{10}$ = saturated or unsaturated, linear or branched, divalent hydrocarbon group comprising from 1 to 30 carbon atoms, n = 5 to 100 and $R_{11}$ = H or $CH_3$;

- N-($C_{8-30}$ alkyl) (meth) acrylamides;
- vinyl esters of formula $R_3-CO-O-CH=CH_2$, where $R_3$ represents a linear or branched alkyl group having from 2 to 12 carbon atoms;
- vinyl compounds of formula $CH_2=CH-R_4$, where $R_4$ is an
- OC(O)-$CH_3$ group, a $C_3$ to $C_8$ cycloalkyl group, a $C_6$ to $C_{20}$ aryl group, a $C_7$ to $C_{30}$ aralkyl group ($C_1$ to $C_9$ alkyl group) or a 4- to 12-membered heterocyclic group comprising one or more heteroatoms chosen from O, N and S, said cycloalkyl, aryl, aralkyl and heterocyclic groups optionally being substituted by one or more substituents chosen from hydroxyl groups, halogen atoms and linear or branched alkyl groups having from 1 to 4 carbon atoms in which one or more heteroatoms chosen from O, N, S and P is/are optionally inserted and said alkyl groups additionally being optionally substituted by one or more substituents chosen from hydroxyl groups and halogen or Si atoms.

**17.** Aerosol device according to Claim 16, **characterized in that** the hydrophobic monomer is chosen from:

- isoprene and butadiene;
- methyl, ethyl, isobutyl, n-butyl, tert-butyl, ethylhexyl, furfuryl, isobornyl, tert-butylcyclohexyl or tert-butylbenzyl acrylates;
- methyl, ethyl, n-butyl, isobutyl, hexyl or ethylhexyl methacrylates;
- N-($C_{6-12}$ alkyl) (meth)acrylamides;
- vinyl esters of formula $R_3-CO-O-CH=CH_2$, where $R_3$ represents a linear or branched alkyl group having from 6 to 12 carbon atoms;
- styrene;
- vinyl acetate and vinylcyclohexane.

**18.** Aerosol device according to any one of the preceding claims, **characterized in that** the compositionally gradient copolymer(s) is or are present in an amount ranging from 0.1 to 20% by weight, preferably from 1 to 17% by weight, with respect to the total weight of the hair treatment composition.

**19.** Aerosol device according to any one of the preceding claims, **characterized in that** the compositionally gradient copolymer is present in the dissolved form or else in the form of an aqueous or organic dispersion.

**20.** Aerosol device according to any one of the preceding claims, **characterized in that** the cosmetically acceptable aqueous medium comprises water and optionally one or more cosmetically acceptable solvents.

**21.** Aerosol device according to Claim 20, **characterized in that** the cosmetically acceptable solvent(s) is or are chosen from lower $C_1$-$C_4$ alcohols, polyols, polyol ethers, acetone and their mixtures.

**22.** Aerosol device according to any one of the preceding claims, **characterized in that** water is present in an amount of between 80 and 99.9% by weight, preferably between 95 and 97% by weight, with respect to the total weight of the hair treatment composition.

**23.** Aerosol device according to any one of the preceding claims, **characterized in that** the hair treatment composition additionally comprises at least one adjuvant chosen from silicones in the soluble, dispersed or microdispersed form, nonionic, anionic, cationic and amphoteric surface-active agents, ceramides and pseudoceramides, vitamins and provitamins, including panthenol, vegetable, animal, mineral and synthetic oils, waxes, water-soluble and fat-soluble sunscreens which may or may not comprise a silicone portion, colored or colorless inorganic and organic pigments, dyes, pearlescent and opacifying agents, sequestering agents, plasticizing agents, solubilizing agents, acidifying agents, basifying agents, inorganic and organic thickening agents, antioxidants, hydroxy acids, penetrating agents, fragrances and preservatives.

**24.** Use of the product vaporized by the aerosol device according to any one of Claims 1 to 23 for the shaping and/or the form retention of the hairstyle.

**25.** Styling process comprising the stage which consists in vaporizing, over wet or dry hair, the hair treatment composition present in the aerosol device according to any one of Claims 1 to 23.

**26.** Use of at least one compositionally gradient copolymer comprising at least two different monomers and exhibiting a weight polydispersity index (PI) of less than or equal to 2.5 in a two-compartment aerosol device comprising a compressed gas chosen from air, nitrogen, carbon dioxide and their mixtures, and optionally a liquefied gas, as propellant for the treatment and/or the shaping of the hairstyle.

**27.** Use according to Claim 26, **characterized in that** the compressed gas is air.

**28.** Use according to Claim 26 or 27, **characterized in that** the pressure of the compressed gas is between 1 and 14 bar.

**29.** Use according to Claim 28, **characterized in that** the pressure of the compressed gas is between 9 and 11 bar.

**30.** Use according to any one of Claims 26 to 29, **characterized in that** the liquefied gas is chosen from hydrocarbons and dimethyl ether.

**31.** Use according to Claim 30, **characterized in that** the hydrocarbons are chosen from $C_{1-5}$ alkanes.

**32.** Use according to any one of the preceding claims, **characterized in that** the liquefied gas is present in an amount ranging from 0 to 95% by weight, preferably between 0 and 60% by weight, with respect to the total weight of the propellant composed of the compressed and liquefied gases.

**33.** Use according to any one of Claims 26 to 32, **characterized in that** the weight polydispersity index (PI) is between 1.1 and 2.3.

**34.** Use according to any one of Claims 26 to 33, **characterized in that** the weight-average molecular weight of the compositionally gradient copolymer is between 5000 and 1 000 000 g/mol.

**35.** Use according to any one of Claims 26 to 34, **characterized in that** the number-average molecular weight of the compositionally gradient copolymer is between 5000 and 1 000 000 g/mol.

**36.** Use according to any one of Claims 26 to 35, **characterized in that** the compositionally gradient copolymer is such that, on the adsorption chromatography (LAC) curve representing the proportion of polymers as a function of the elution volume, the difference ($V^{1/2}$ max - $V^{1/2}$ min) is less than or equal to 3.5, preferably between 1 and 2.8, "$V^{1/2}$ min" being the minimum value of the elution volume at mid-height of the curve and "$V^{1/2}$ max" being the maximum value of the elution volume at mid-height of the curve.

**37.** Use according to any one of Claims 26 to 36, **characterized in that** at least one of the monomers of the compositionally gradient copolymer is a hydrophilic monomer.

**38.** Use according to Claim 37, **characterized in that** the hydrophilic monomer is chosen from:

- amino($C_1$-$C_4$ alkyl) (meth)acrylate derivatives;
- di($C_1$-$C_8$ alkyl)allylamines;
- vinylamine;
- vinylpyridines;

and their salts with inorganic acids or with organic acids, or their quaternized forms;

- ethylenic carboxylic acids comprising from 3 to 20 carbon atoms, or their salts;
- carboxylic anhydrides carrying a vinyl double bond and comprising from 4 to 30 carbon atoms;
- sulfonic or phosphonic ethylenic acids, and their salts;
- vinyl alcohol;
- acrylamide or methacrylamide, N-($C_1$-$C_6$alkyl)(meth)-acrylamides, N,N-di($C_1$-$C_3$alkyl)(meth)acrylamides or N, N-di($C_1$-$C_4$alkyl)amino($C_1$-$C_6$alkyl)(meth)acrylamides;
- hydroxy($C_2$-$C_4$alkyl)(meth)acrylates;
- polyethylene glycol (5 to 100 ethylene oxide units) or glycol (meth)acrylates which are or are not substituted on their terminal functional group by $C_1$-$C_4$ alkyl, phosphate, phosphonate or sulfonate groups;
- $C_1$-$C_4$ alkoxyalkyl (meth)acrylates;
- polysaccharide (meth)acrylates;
- optionally cyclic vinylamides;
- vinyl ethers;
- methacrylamidopropoxytrimethylammonium;
- N,N-dimethyl-N-methacryloyloxyethyl-N-(3-sulfopropyl)-ammonium;
- 3-methacryloylethoxycarbonylpyridinium;
- the compound of formula:

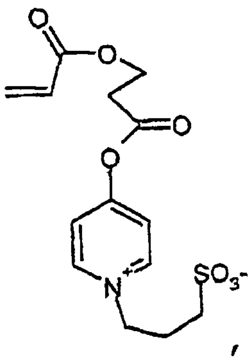
,

and
- N-(3-sulfopropyl)-4-vinylpyridinium of formula:

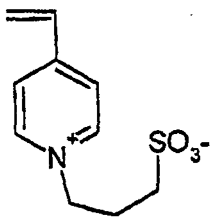
.

**39.** Use according to Claim 38, **characterized in that** the hydrophilic monomer is chosen from N,N-dimethylaminoethyl methacrylate (MADAME), acrylic acid, methacrylic acid, crotonic acid, styrenesulfonic acid, acrylamidopropanesulfonic acid, dimethylaminopropylmethacrylamide (DMAPMA), styrenesulfonate, hydroxyethyl acrylate, glyceryl acrylate, methoxyethyl (meth)acrylate, ethoxyethyl (meth)acrylate, methoxypolyethylene glycol (8 or 12 EO) (meth) acrylate, hydroxypolyethylene glycol (meth)acrylate, N-vinylpyrrolidone, N-vinylcaprolactam, acrylamide and N,N-dimethylacrylamide.

**40.** Use according to any one of Claims 26 to 39, **characterized in that** at least one of the monomers of the compositionally gradient copolymer is a hydrophobic monomer.

**41.** Use according to Claim 40, **characterized in that** the hydrophobic monomer is chosen from:

- ethylenic hydrocarbons comprising from 2 to 30 carbon atoms;
- acrylates of formula $CH_2=CHCOOR_1$, in which $R_1$ represents a saturated or unsaturated, linear, branched or cyclic, hydrocarbon group comprising from 1 to 30 carbon atoms in which one or more heteroatoms chosen from O, N, S and Si is/are optionally inserted, said hydrocarbon group additionally being optionally substituted by one or more substituents chosen from hydroxyl groups and halogen atoms,

or $R_1$ represents a $-(R_{10})_x-(OC_2H_4)_n-OR_{11}$ group, with x = 0 or 1, $R_{10}$ = saturated or unsaturated, linear or branched, divalent hydrocarbon group comprising from 1 to 30 carbon atoms, n = 5 to 100 and $R_{11}$ = H or $CH_3$;

- methacrylates of formula $CH_2=C(CH_3)-COOR_2$, in which $R_2$ represents a saturated or unsaturated, linear, branched or cyclic, hydrocarbon group comprising from 1 to 30 carbon atoms in which one or more heteroatoms chosen from O, N, S and Si is/are optionally inserted, said hydrocarbon group additionally being optionally substituted by one or more substituents chosen from hydroxyl groups and halogen atoms,

or $R_2$ represents $-(R_{10})_x-(OC_2H_4)_n-OR_{11}$, with x = 0 or 1, $R_{10}$ = saturated or unsaturated, linear or branched, divalent hydrocarbon group comprising from 1 to 30 carbon atoms, n = 5 to 100 and $R_{11}$ = H or $CH_3$ ;

- N-($C_{8-30}$alkyl)(meth)acrylamides;
- vinyl esters of formula $R_3-CO-O-CH=CH_2$, where $R_3$ represents a linear or branched alkyl group having from 2 to 12 carbon atoms;
- vinyl compounds of formula $CH_2=CH-R_4$, where $R_4$ is an
- $OC(O)-CH_3$ group, a $C_3$ to $C_8$ cycloalkyl group, a $C_6$ to $C_{20}$ aryl group, a $C_7$ to $C_{30}$ aralkyl group ($C_1$ to $C_4$ alkyl group) or a 4- to 12-membered heterocyclic group comprising one or more heteroatoms chosen from O, N and S, said cycloalkyl, aryl, aralkyl and heterocyclic groups optionally being substituted by one or more substituents chosen from hydroxyl groups, halogen atoms and linear or branched alkyl groups having from 1 to 4 carbon atoms in which one or more heteroatoms chosen from O, N, S and P is/are optionally inserted and said alkyl groups additionally being optionally substituted by one or more substituents chosen from hydroxyl groups and halogen or Si atoms.

**42.** Use according to Claim 41, **characterized in that** the hydrophobic monomer is chosen from:

- isoprene and butadiene;
- methyl, ethyl, isobutyl, n-butyl, tert-butyl, ethylhexyl, furfuryl, isobornyl, tert-butylcyclohexyl or tert-butylbenzyl acrylates;
- methyl, ethyl, n-butyl, isobutyl, hexyl or ethylhexyl methacrylates;
- N-($C_{6-12}$alkyl)(meth)acrylamides;
- vinyl esters of formula $R_3-CO-O-CH=CH_2$, where $R_3$ represents a linear or branched alkyl group having from 6 to 12 carbon atoms;
- styrene;
- vinyl acetate and vinylcyclohexane.

**43.** Use according to any one of Claims 26 to 42, **characterized in that** the compositionally gradient copolymer is present in the dissolved form or else in the form of an aqueous or organic dispersion.

## Patentansprüche

**1.** Aerosolvorrichtung mit zwei Abteilungen, enthaltend:

(a) in einer ersten Abteilung eine Zusammensetzung für die Haarbehandlung, die in einem kosmetisch akzeptablen, wässrigen Medium mindestens ein Copolymer mit gradierter Zusammensetzung enthält, das mindestens zwei unterschiedliche Monomere enthält und einen Polydispersitätsindex (Ip) von 2,5 oder darunter aufweist, und
(b) in einer zweiten Abteilung ein Druckgas, das unter Luft, Stickstoff, Kohlendioxid und deren Gemischen

ausgewählt ist, und gegebenenfalls mindestens ein Flüssiggas.

**2.** Aerosolvorrichtung nach Anspruch 1, **dadurch gekennzeichnet, dass** das Druckgas Luft ist.

**3.** Aerosolvorrichtung nach Anspruch 1 oder 2, **dadurch gekennzeichnet, dass** der Druck des Druckgases im Bereich von 1 bis 14 bar liegt.

**4.** Aerosolvorrichtung nach Anspruch 3, **dadurch gekennzeichnet, dass** der Druck des Druckgases im Bereich von 9 bis 11 bar liegt.

**5.** Aerosolvorrichtung nach einem der vorhergehenden Ansprüche, **dadurch gekennzeichnet, dass** das Flüssiggas unter den Kohlenwasserstoffen und Dimethylether ausgewählt ist.

**6.** Aerosolvorrichtung nach Anspruch 5, **dadurch gekennzeichnet, dass** die Kohlenwasserstoffe unter den $C_{1-5}$-Alkanen ausgewählt sind.

**7.** Aerosolvorrichtung nach einem der vorhergehenden Ansprüche, **dadurch gekennzeichnet, dass** das Flüssiggas in einer Menge von 0 bis 95 Gew.-%, vorzugsweise 0 bis 60 Gew.-%, bezogen auf das Gesamtgewicht des Treibmittels, das aus Druckgas und Flüssiggas besteht, enthalten ist.

**8.** Aerosolvorrichtung nach einem der vorhergehenden Ansprüche, **dadurch gekennzeichnet, dass** der Polydispersitätsindex (Ip) im Bereich von 1,1 bis 2,3 liegt.

**9.** Aerosolvorrichtung nach einem der vorhergehenden Ansprüche, **dadurch gekennzeichnet, dass** die gewichtsmittlere Molmasse des Copolymers mit gradierter Zusammensetzung im Bereich von 5 000 bis 1 000 000 g/mol liegt.

**10.** Aerosolvorrichtung nach einem der vorhergehenden Ansprüche, **dadurch gekennzeichnet, dass** die zahlenmittlere Molmasse des Copolymers mit gradierter Zusammensetzung im Bereich von 5 000 bis 1 000 000 g/mol liegt.

**11.** Aerosolvorrichtung nach einem der vorhergehenden Ansprüche, **dadurch gekennzeichnet, dass** das Copolymer mit gradierter Zusammensetzung so vorliegt, dass in der Adsorptionschromatographiekurve (CAL), die den Anteil der Polymere in Abhängigkeit des Elutionsvolumens angibt, der Unterschied ($V^{1/2}$ max - $V^{1/2}$ min) 3,5 oder darunter beträgt und vorzugsweise im Bereich von 1 bis 2,8 liegt, wobei "$V^{1/2}$ min" der Minimalwert des Elutionsvolumens bei halber Höhe der Kurve und "$V^{1/2}$ max" der Maximalwert des Elutionsvolumens bei halber Höhe der Kurve ist.

**12.** Aerosolvorrichtung nach einem der vorhergehenden Ansprüche, **dadurch gekennzeichnet, dass** mindestens eines der Monomere des Copolymers mit gradierter Zusammensetzung ein hydrophiles Monomer ist.

**13.** Aerosolvorrichtung nach Anspruch 12, **dadurch gekennzeichnet, dass** das hydrophile Monomer ausgewählt ist unter:

- Derivaten von Aminoalkyl $C_{1-4}$ (meth)acrylaten;
- $C_{1-8}$-Dialkylallylaminen;
- Vinylamin;
- Vinylpyridinen;

sowie ihren Salzen mit anorganischen Säuren oder einer organischen Säure oder ihren quaternisierten Formen;

- ethylenischen Carbonsäuren mit 3 bis 20 Kohlenstoffatomen oder deren Salzen;
- Carbonsäureanhydriden, die eine Vinyldoppelbindung tragen und 4 bis 30 Kohlenstoffatome aufweisen;
- ethylenischen Sulfonsäuren oder Phosphonsäuren und deren Salzen;
- Vinylalkohol;
- Acrylamid oder Methacrylamid, N-Alkyl($C_{1-6}$)(meth)acrylamiden, N,N-Dialkyl($C_{1-3}$)(meth)acrylamiden, N,N-Dialkyl($C_{1-4}$)aminoalkyl($C_{1-6}$)(meth)acrylamiden;
- Hydroxyalkyl($C_{2-4}$)(meth)acrylaten;
- Polyethylenglycol(meth)acrylaten (5 bis 100 Ethylenoxideinheiten) oder Glycol(meth)acrylaten, die unsubstituiert sind oder an ihrer endständigen Funktion mit $C_{1-4}$-Alkylgruppen, Phosphat, Phosphonat, Sulfonat substituiert sind;
- Alkoxyalkyl($C_{1-4}$)(meth)acrylaten;
- Polysaccharid(meth)acrylaten;

- Vinylamiden, die gegebenenfalls cyclisch sind;
- Vinylethern;
- Methacrylamidopropoxy-trimethylammonium;
- N,N-Dimethyl-N-methacryloxyethyl-N-(3-sulfopropyl)-ammonium;
- 3-Methacryloylethoxycarbonylpyridinium;
- der Verbindung der Formel:

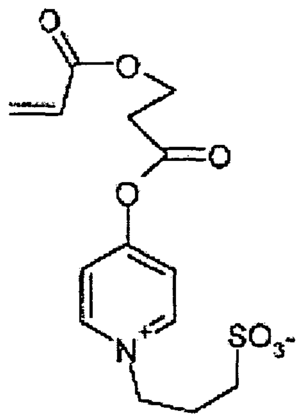

, und
- dem N-(3-Sulfopropyl)-4-vinylpyridinium der Formel:

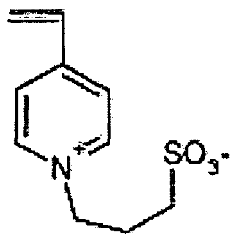

**14.** Aerosolvorrichtung nach Anspruch 13, **dadurch gekennzeichnet, dass** das hydrophile Monomer unter N,N-Dimethylaminoethylmethacrylat (MADAME), Acrylsäure, Methacrylsäure, Crotonsäure, Styrolsulfonsäure, Acrylamidopropansulfonsäure, Dimethylaminopropylmethacrylamid (DMAPMA); Styrolsulfonat, Hydroxyethylacrylat, Glycerylacrylat, Methoxyethyl(meth)acrylat, Ethoxyethyl(meth)acrylat, Methoxypolyethylenglycol(meth)acrylat (8 oder 12 EO); Hydroxypolyethylenglycol(meth)acrylat; N-Vinylpyrrolidon, N-Vinylcaprolactam, Acrylamid und N,N-Dimethylacrylamid ausgewählt ist.

**15.** Aerosolvorrichtung nach einem der vorhergehenden Ansprüche, **dadurch gekennzeichnet, dass** mindestens eines der Monomere des Copolymers mit gradierter Zusammensetzung ein hydrophobes Monomer ist.

**16.** Aerosolvorrichtung nach Anspruch 15, **dadurch gekennzeichnet, dass** das hydrophobe Monomer ausgewählt ist unter:

- ethylenischen Kohlenwasserstoffen mit 2 bis 30 Kohlenstoffatomen;
- Acrylaten der Formel $CH_2$=$CHCOOR_1$, wobei $R_1$ eine gesättigte oder ungesättigte Kohlenwasserstoffgruppe mit 1 bis 30 Kohlenstoffatomen ist, die linear, verzweigt oder cyclisch ist und bei der gegebenenfalls ein oder mehrer Heteroatome, die unter O, N, S und Si ausgewählt sind, in der Kette vorliegen, wobei die Kohlenwasserstoffgruppe ferner gegebenenfalls mit einem oder mehreren Substituenten substituiert ist, die unter den Hydroxygruppen und Halogenatomen ausgewählt sind, oder $R_1$ bedeutet eine Gruppe $-(R_{10})_x-(OC_2H_4)_n-OR_{11}$, mit x = 0 oder 1, $R_{10}$ = eine gesättigte oder ungesättigte, geradkettige oder verzweigte zweiwertige Kohlenwasserstoffgruppe, die 1 bis 30 Kohlenstoffatome enthält, n = 5 bis 100 und $R_{11}$ = H oder $CH_3$;

- Methacrylaten der Formel: $CH_2=C(CH_3)-COOR_2$, worin $R_2$ eine gesättigte oder ungesättigte Kohlenwasserstoffgruppe mit 1 bis 30 Kohlenstoffatomen ist, die linear, verzweigt oder cyclisch ist und bei der gegebenenfalls ein oder mehrere Heteroatome, die unter O, N, S und Si ausgewählt sind, in der Kette vorliegen, wobei die Kohlenwasserstoffgruppe ferner gegebenenfalls mit einem oder mehreren Substituenten substituiert ist, die unter Hydroxygruppen und Halogenatomen ausgewählt sind,
oder $R_2$ bedeutet $-(R_{10})_x-(OC_2H_4)_n-OR_{11}$ mit x = 0 oder 1, $R_{10}$ = eine gesättigte oder ungesättigte, geradkettige oder verzweigte zweiwertige Kohlenwasserstoffgruppe, die 1 bis 30 Kohlenstoffatome enthält, n = 5 bis 100 und $R_{11}$ = H oder $CH_3$;
- N-Alkyl($C_{8-30}$)(meth)acrylamiden ;
- Vinylestern der Formel: $R_3-CO-O-CH=CH_2$, wobei $R_3$ eine lineare oder verzweigte Alkylgruppe mit 2 bis 12 Kohlenstoffatomen ist;
- Vinylverbindungen der Formel: $CH_2=CH-R_4$, wobei $R_4$ eine Gruppe-$OC(O)-CH_3$, eine $C_{3-8}$-Cycloalkylgruppe; eine $C_{6-20}$-Arylgruppe; eine $C_{7-30}$-Aralkylgruppe ($C_{1-4}$-Alkylgruppe); eine heterocyclische Gruppe mit 4 bis 12 Bestandteilen, die ein oder mehrere Heteroatome enthält, die unter O, N und S ausgewählt sind; wobei die Cycloalkylgruppe, Arylgruppe, Aralkylgruppe und heterocyclische Gruppe gegebenenfalls mit einem oder mehreren Substituenten substituiert ist, die unter den Hydroxygruppen, Halogenatomen und Alkylgruppen mit 1 bis 4 Kohlenstoffatomen ausgewählt sind, die linear oder verzweigt sind und bei denen gegebenenfalls ein oder mehrere Heteroatome, die unter O, N, S und P ausgewählt sind, in der Kette vorhanden sind, wobei die Alkylgruppen ferner gegebenenfalls mit einem oder mehreren Substituenten substituiert sind, die unter den Hydroxygruppen und den Halogenatomen oder Si ausgewählt sind.

**17.** Aerosolvorrichtung nach Anspruch 16, **dadurch gekennzeichnet, dass** das hydrophobe Monomer ausgewählt ist unter:

- Isopren und Butadien;
- Methylacrylat, Ethylacrylat, Isobutylacrylat, n-Butylacrylat, *tert*-Butylacrylat, Ethylhexylacrylat, Furfurylacrylat, Isobornylacrylat, *tert*-Butylcyclohexylacrylat, *tert*-Butylbenzylacrylat;
- Methylmethacrylat, Ethylmethacrylat, n-Butylmethacrylat, Isobutylmethacrylat, Hexylmethacrylat, Ethylhexylmethacrylat;
- N-(Alkyl($C_{6-12}$)(meth)acrylamiden;
- Vinylestern der Formel: $R_3-CO-O-CH=CH_2$, wobei $R_3$ eine Alkylgruppe mit 6 bis 12 Kohlenstoffatomen bedeutet, die geradkettig oder verzweigt ist;
- Styrol;
- Vinylacetat und Vinylcyclohexan.

**18.** Aerosolvorrichtung nach einem der vorhergehenden Ansprüche, **dadurch gekennzeichnet, dass** das Copolymer (oder die Copolymere) mit gradierter Zusammensetzung in einer Menge von 0,1 bis 20 Gew.-% und vorzugsweise 1 bis 17 Gew.-%, bezogen auf das Gesamtgewicht der Zusammensetzung für die Haarbehandlung, enthalten ist (sind).

**19.** Aerosolvorrichtung nach einem der vorhergehenden Ansprüche, **dadurch gekennzeichnet, dass** das Copolymer mit gradierter Zusammensetzung in solubilisierter Form oder in Form einer wässrigen oder organischen Dispersion vorliegt.

**20.** Aerosolvorrichtung nach einem der vorhergehenden Ansprüche, **dadurch gekennzeichnet, dass** das kosmetisch akzeptable, wässrige Medium Wasser und gegebenenfalls ein oder mehrere kosmetisch akzeptable Lösungsmittel enthält.

**21.** Aerosolvorrichtung nach Anspruch 20, **dadurch gekennzeichnet, dass** das oder die kosmetisch akzeptable(n) Lösungsmittel unter den niederen $C_{1-4}$-Alkoholen, Polyolen, Polyolethern, Aceton und deren Gemischen ausgewählt ist (sind).

**22.** Aerosolvorrichtung nach einem der vorhergehenden Ansprüche, **dadurch gekennzeichnet, dass** das Wasser in einer Menge von 80 bis 99,9 % und vorzugsweise 95 bis 97 Gew.-%, bezogen auf das Gesamtgewicht der Zusammensetzung für die Haarbehandlung, enthalten ist.

**23.** Aerosolvorrichtung nach einem der vorhergehenden Ansprüche, **dadurch gekennzeichnet, dass** die Zusammensetzung für die Haarbehandlung ferner mindestens einen Zusatzstoff enthält, der unter den Siliconen in gelöster,

dispergierter, mikrodispergierter Form, nichtionischen, anionischen, kationischen und amphoteren grenzflächenaktiven Stoffen, Ceramiden und Pseudoceramiden, Vitaminen und Provitaminen, darunter Panthenol, pflanzlichen, tierischen, mineralischen und synthetischen Ölen, Wachsen, Ceramiden und Pseudoceramiden, wasserlöslichen und fettlöslichen Sonnenschutzfiltern, die siliconiert oder nichtsiliconiert sind, anorganischen und organischen, farbigen oder nicht farbigen Pigmenten, Farbstoffen, Perlglanzmitteln und Trübungsmitteln, Maskierungsmitteln, Weichmachern, Solubilisierungsmitteln, Ansäuerungsmitteln, Alkalisierungsmitteln, anorganischen und organischen Verdickungsmitteln, Antioxidantien, Hydroxysäuren, Penetrationsmitteln, Parfums und Konservierungsmitteln ausgewählt ist.

**24.** Verwendung des durch die Aerosolvorrichtung nach einem der Ansprüche 1 bis 23 zerstäubten Produkts für die Formgebung und/oder Festigung der Frisur.

**25.** Verfahren für die Frisurengestaltung, das einen Schritt umfasst, der darin besteht, die in der Aerosolvorrichtung nach einem der Ansprüche 1 bis 23 enthaltene Zusammensetzung für die Haarbehandlung auf den feuchten oder nicht feuchten Haaren zu zerstäuben.

**26.** Verwendung mindestens eines Copolymers mit gradierter Zusammensetzung, das mindestens zwei unterschiedliche Monomere enthält und einen Polydispersitätsindex (Ip) von höchstens 2,5 aufweist, in einer Aerosolvorrichtung mit zwei Abteilungen, die als Treibmittel ein Druckgas, das unter Luft, Stickstoff, Kohlendioxid und deren Gemischen ausgewählt ist, und gegebenenfalls ein Flüssiggas enthält, für die Behandlung und/oder Formgebung der Frisur.

**27.** Verwendung nach Anspruch 26, **dadurch gekennzeichnet, dass** das Druckgas Luft ist.

**28.** Verwendung nach Anspruch 26 oder 27, **dadurch gekennzeichnet, dass** der Druck des Druckgases im Bereich von 1 bis 14 bar liegt.

**29.** Verwendung nach Anspruch 28, **dadurch gekennzeichnet, dass** der Druck des Druckgases im Bereich von 9 bis 11 bar liegt.

**30.** Verwendung nach einem der Ansprüche 26 bis 29, **dadurch gekennzeichnet, dass** das Flüssiggas unter den Kohlenwasserstoffen und Dimethylether ausgewählt ist.

**31.** Verwendung nach Anspruch 30, **dadurch gekennzeichnet, dass** die Kohlenwasserstoffe unter den $C_{1-5}$-Alkanen ausgewählt sind.

**32.** Verwendung nach einem der vorhergehenden Ansprüche, **dadurch gekennzeichnet, dass** das Flüssiggas in einer Menge von 0 bis 95 Gew.-% und vorzugsweise 0 bis 60 Gew.-%, bezogen auf das Gesamtgewicht des Treibmittels, das aus dem Druckgas und dem Flüssiggas besteht, enthalten ist.

**33.** Verwendung nach einem der Ansprüche 26 bis 32, **dadurch gekennzeichnet, dass** der Polydispersitätsindex (Ip) im Bereich von 1,1 bis 2,3 liegt.

**34.** Verwendung nach einem der Ansprüche 26 bis 33, **dadurch gekennzeichnet, dass** die gewichtsmittlere Molmasse des Copolymers mit gradierter Zusammensetzung im Bereich von 5 000 bis 1 000 000 g/mol liegt.

**35.** Verwendung nach einem der Ansprüche 26 bis 34, **dadurch gekennzeichnet, dass** die zahlenmittlere Molmasse des Copolymers mit gradierter Zusammensetzung im Bereich von 5 000 bis 1 000 000 g/mol liegt.

**36.** Verwendung nach einem der Ansprüche 26 bis 35, **dadurch gekennzeichnet, dass** das Copolymer mit gradierter Zusammensetzung so vorliegt, dass in der Adsorptionschromatographiekurve (CAL), die den Anteil der Polymere in Abhängigkeit des Elutionsvolumens angibt, der Unterschied ($V^{1/2}$ max - $V^{1/2}$ min) 3,5 oder darunter beträgt und vorzugsweise im Bereich von 1 bis 2,8 liegt, wobei "$V^{1/2}$ min" der Minimalwert des Elutionsvolumens bei halber Höhe der Kurve und "$V^{1/2}$ max" der Maximalwert des Elutionsvolumens bei halber Höhe der Kurve ist.

**37.** Verwendung nach einem der Ansprüche 26 bis 36, **dadurch gekennzeichnet, dass** mindestens eines der Monomere des Copolymers mit gradierter Zusammensetzung ein hydrophiles Monomer ist.

**38.** Verwendung nach Anspruch 37, **dadurch gekennzeichnet, dass** das hydrophile Monomer ausgewählt ist unter:

- Derivaten von Aminoalkyl $C_{1-4}$(meth)acrylaten;
- $C_{1-8}$-Dialkylallylaminen;
- Vinylamin;
- Vinylpyridinen;

sowie ihren Salzen mit anorganischen Säuren oder einer organischen Säure oder ihren quaternisierten Formen;

- ethylenischen Carbonsäuren mit 3 bis 20 Kohlenstoffatomen oder deren Salzen;
- Carbonsäureanhydriden, die eine Vinyldoppelbindung tragen und 4 bis 30 Kohlenstoffatome aufweisen;
- ethylenischen Sulfonsäuren oder Phosphonsäuren und deren Salzen;
- Vinylalkohol;
- Acrylamid oder Methacrylamid, N-Alkyl($C_{1-6}$)(meth)acrylamiden, N,N-Dialkyl($C_{1-3}$)(meth)acrylamiden, N,N-Dialkyl($C_{1-4}$)aminoalkyl($C_{1-6}$)(meth)acrylamiden;
- Hydroxyalkyl($C_{2-4}$)(meth)acrylaten;
- Polyethylenglycol(meth)acrylaten (5 bis 100 Ethylenoxideinheiten) oder Glycol(meth)acrylaten, die unsubstituiert sind oder an ihrer endständigen Funktion mit $C_{1-4}$ Alkylgruppen, Phosphat, Phosphonat, Sulfonat substituiert sind;
- Alkoxyalkyl($C_{1-4}$)(meth)acrylaten;
- Polysaccharid(meth)acrylaten;
- Vinylamiden, die gegebenenfalls cyclisch sind;
- Vinylethern;
- Methacrylamidopropoxy-trimethylammonium;
- N,N-Dimethyl-N-methacryloxyethyl-N-(3-sulfopropyl)-ammonium;
- 3-Methacryloylethoxycarbonylpyridinium;
- der Verbindung der Formel:

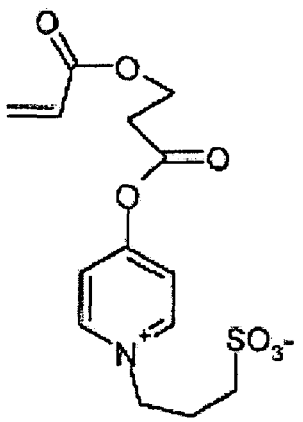

,

und

- dem N-(3-Sulfopropyl)-4-vinylpyridinium der Formel:

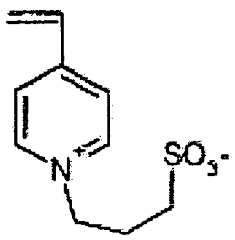

**39.** Verwendung nach Anspruch 38, **dadurch gekennzeichnet, dass** das hydrophile Monomer unter N,N-Dimethylaminoethylmethacrylat (MADAME), Acrylsäure, Methacrylsäure, Crotonsäure, Styrolsulfonsäure, Acrylamidopropansulfonsäure, Dimethylaminopropylmethacrylamid (DMAPMA); Styrolsulfonat, Hydroxyethylacrylat, Glycerylacrylat, Methoxyethyl(meth)acrylat, Ethoxyethyl(meth)acrylat, Methoxypolyethylenglycol(meth)acrylat (8 oder 12 EO); Hydroxypolyethylenglycol(meth)acrylat; N-Vinylpyrrolidon, N-Vinylcaprolactam, Acrylamid und N,N-Dimethylacrylamid ausgewählt ist.

**40.** Verwendung nach einem der Ansprüche 26 bis 39, **dadurch gekennzeichnet, dass** mindestens eines der Monomere des Copolymers mit gradierter Zusammensetzung ein hydrophobes Monomer ist.

**41.** Verwendung nach Anspruch 40, **dadurch gekennzeichnet, dass** das hydrophobe Monomer ausgewählt ist unter:

- ethylenischen Kohlenwasserstoffen mit 2 bis 30 Kohlenstoffatomen;
- Acrylaten der Formel $CH_2=CHCOOR_1$, wobei $R_1$ eine gesättigte oder ungesättigte Kohlenwasserstoffgruppe mit 1 bis 30 Kohlenstoffatomen ist, die linear, verzweigt oder cyclisch ist und bei der gegebenenfalls ein oder mehrer Heteroatome, die unter O, N, S und Si ausgewählt sind, in der Kette vorliegen, wobei die Kohlenwasserstoffgruppe ferner gegebenenfalls mit einem oder mehreren Substituenten substituiert ist, die unter den Hydroxygruppen und Halogenatomen ausgewählt sind, oder $R_1$ bedeutet eine Gruppe $-(R_{10})_x-(OC_2H_4)_n-OR_{11}$, mit x = 0 oder 1, $R_{10}$ = eine gesättigte oder ungesättigte, geradkettige oder verzweigte zweiwertige Kohlenwasserstoffgruppe, die 1 bis 30 Kohlenstoffatome enthält, n = 5 bis 100 und $R_{11}$ = H oder $CH_3$;
- Methacrylaten der Formel: $CH_2=C(CH_3)-COOR_2$, worin $R_2$ eine gesättigte oder ungesättigte Kohlenwasserstoffgruppe mit 1 bis 30 Kohlenstoffatomen ist, die linear, verzweigt oder cyclisch ist und bei der gegebenenfalls ein oder mehrere Heteroatome, die unter O, N, S und Si ausgewählt sind, in der Kette vorliegen, wobei die Kohlenwasserstoffgruppe ferner gegebenenfalls mit einem oder mehreren Substituenten substituiert ist, die unter Hydroxygruppen und Halogenatomen ausgewählt sind,
oder $R_2$ bedeutet $-(R_{10})_x-(OC_2H_4)_n-OR_{11}$ mit x = 0 oder 1, $R_{10}$ = eine gesättigte oder ungesättigte, geradkettige oder verzweigte zweiwertige Kohlenwasserstoffgruppe, die 1 bis 30 Kohlenstoffatome enthält, n = 5 bis 100 und $R_{11}$ = H oder $CH_3$;
- N-Alkyl($C_{8-30}$)(meth)acrylamiden;
- Vinylestern der Formel: $R_3-CO-O-CH=CH_2$, wobei $R_3$ eine lineare oder verzweigte Alkylgruppe mit 2 bis 12 Kohlenstoffatomen ist;
- Vinylverbindungen der Formel: $CH_2=CH-R_4$, wobei $R_4$ eine Gruppe-$OC(O)-CH_3$, eine $C_{3-8}$-Cycloalkylgruppe; eine $C_{6-20}$-Arylgruppe; eine $C_{7-30}$-Aralkylgruppe ($C_{1-4}$-Alkylgruppe); eine heterocyclische Gruppe mit 4 bis 12 Bestandteilen, die ein oder mehrere Heteroatome enthält, die unter O, N und S ausgewählt sind; wobei die Cycloalkylgruppe, Arylgruppe, Aralkylgruppe und heterocyclische Gruppe gegebenenfalls mit einem oder mehreren Substituenten substituiert ist, die unter den Hydroxygruppen, Halogenatomen und Alkylgruppen mit 1 bis 4 Kohlenstoffatomen ausgewählt sind, die linear oder verzweigt sind und bei denen gegebenenfalls ein oder mehrere Heteroatome, die unter O, N, S und P ausgewählt sind, in der Kette vorhanden sind, wobei die Alkylgruppen ferner gegebenenfalls mit einem oder mehreren Substituenten substituiert sind, die unter den Hydroxygruppen und den Halogenatomen oder Si ausgewählt sind.

**42.** Verwendung nach Anspruch 41, **dadurch gekennzeichnet, dass** das hydrophobe Monomer ausgewählt ist unter:

- Isopren und Butadien;
- Methylacrylat, Ethylacrylat, Isobutylacrylat, *n*-Butylacrylat, *tert*-Butylacrylat, Ethylhexylacrylat, Furfurylacrylat, Isobornylacrylat, *tert*-Butylcyclohexylacrylat, *tert*-Butylbenzylacrylat;
- Methylmethacrylat, Ethylmethacrylat, *n*-Butylmethacrylat, Isobutylmethacrylat, Hexylmethacrylat, Ethylhexylmethacrylat;
- N-Alkyl($C_{6-12}$)(meth)acrylamiden;
- Vinylestern der Formel: $R_3-CO-O-CH=CH_2$, wobei $R_3$ eine Alkylgruppe mit 6 bis 12 Kohlenstoffatomen bedeutet, die geradkettig oder verzweigt ist;
- Styrol;
- Vinylacetat und Vinylcyclohexan.

**43.** Verwendung nach einem der Ansprüche 26 bis 42, **dadurch gekennzeichnet, dass** das Copolymer mit gradierter Zusammensetzung in solubilisierter Form oder in Form einer wässrigen oder organischen Dispersion vorliegt.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1444974 A
- FR 2848429
- WO 0071607 A
- WO 2004055071 A
- WO 9624620 A
- WO 0071501 A
- WO 9630421 A
- FR 2821620
- WO 9801478 A
- WO 9935177 A
- WO 9858974 A
- WO 9931144 A


### Littérature non-brevet citée dans la description


- **FISCHER.** *Chemical Reviews,* 2001, vol. 101, 3581
- **TORDO ; GNANOU.** *J. Am. Chem. Soc.,* 2000, vol. 122, 5929
- **HAWKER.** *J. Am. Chem. Soc.,* 1999, vol. 121, 3904
- **RIZZARDO.** *Macromolecules,* 1998, vol. 31, 5559
- **T. PAKULA et al.** *Macromol. Theory Simul.,* 1996, vol. 5, 987-1006
- **A. AKSIMETIEV et al.** *J. of Chem. Physics,* vol. 111 (5
- **M. JANCO.** *J. Polym. Sci., Part A: Polym. Chem.,* 2000, vol. 38 (15), 2767-2778
- **M. ZAREMSKI et al.** *Macromolecules,* 2000, vol. 33 (12), 4365-4372
- **K. MATYJASZEWSKI.** *J. Phys. Org. Chem.,* 2000, vol. 13 (12), 775-786
- **GRAY.** *Polym. Prepr. (Am. Chem. Soc., Div. Polym. Chem.,* 2001, vol. 42 (2), 337-338
- **K. MATYJASZEWSKI.** *Chem. Rev.,* 2001, vol. 101 (9), 2921-2990